# FASCICULE DE BREVET EUROPEEN

(11) **EP 4 025 358 B1**
(45) Date de publication et mention de la délivrance du brevet: **02.07.2025**
(21) Numéro de dépôt: 20764419.6
(22) Date de dépôt: 04.09.2020
(51) Int. Cl.: B29B 17/02, B01D 11/04, B09B 3/00, B09B 3/40, B09B 5/00, B29K 105/04, B09B 101/02

(54) **PROCÉDÉ DE RÉCUPÉRATION ET DE SÉPARATION D'HYDROCARBURES FLUORÉS INSATURÉS**
VERFAHREN ZUR RÜCKGEWINNUNG UND ABTRENNUNG VON UNGESÄTTIGTEN FLUORIERTEN KOHLENWASSERSTOFFEN
METHOD FOR RECOVERING AND SEPARATING UNSATURATED FLUORINATED HYDROCARBONS

(30) Priorité: 06.09.2019 FR 1909815
(43) Date de publication de la demande: 13.07.2022
(73) Titulaire: ARKEMA FRANCE, 92800 Puteaux (FR)
(72) Inventeur: DUBOIS, Jean-Luc, 92705 Colombes Cedex (FR)
(74) Mandataire: Arkema Patent
(86) Numéro de dépôt international: PCT/EP2020/074783
(87) Numéro de publication internationale: WO 2021/043991

(56) Documents cités:
- EP-A2- 0 392 760
- EP-A2- 0 514 106
- EP-A2- 0 636 429
- WO-A1-2014/193689
- WO-A1-93/22077
- WO-A2-02/02209
- CN-A- 106 807 723
- US-A1- 2006 200 964
- US-A1- 2012 059 200

## Description

### Domaine technique de l'invention

La présente invention concerne un procédé de récupération et de séparation d'hydrocarbures fluorés insaturés. En particulier, la présente invention concerne un procédé de récupération et de séparation d'hydrocarbures fluorés insaturés contenus dans des mousses isolantes.

### Arrière-plan technologique de l'invention

La mise au rebut ou le recyclage de réfrigérateurs implique l'enlèvement des déchets encombrants et leur livraison dans des usines de retraitement spécifiques. La mise à la ferraille de ce type de dispositif est connue notamment de DE2850130. Cela implique notamment un concassage des différents éléments, leur séparation puis le recyclage des différents matériaux si possible. Des procédés de recyclage de mousses avec récupération d'agents gonflants sont décrits dans les documents EP 0 392 760 A2, WO 93/22077 A1, US 2006/200964 A1, EP 0 514 106 A2, WO 02/02209 A2, EP 0 636 429 A2 et CN 106 807 723 A.

Les réfrigérateurs contiennent notamment des mousses isolantes faites généralement d'un polymère à base de polyuréthane. Ces mousses contiennent également des agents gonflants. Les compositions d'agents gonflants utilisées comprennent notamment des hydrohalocarbures. La législation autour des dérivés hydrohalocarbures ayant évolué au cours du temps, les dispositifs contenant des mousses isolantes peuvent contenir des CFC (chlorofluorocarbures), des HFC (hydrofluorocarbures) ou des HFO (hydrofluorooléfines). En effet, les hydrofluorooléfines attirent l'attention parce qu'elles offrent un comportement prometteur à faible potentiel de réchauffement climatique global. Il existe donc un besoin pour traiter et recycler des dispositifs contenant des mousses isolantes contenant les nouvelles générations d'agents gonflants mais également un besoin pour traiter et recycler des dispositifs contenant des mousses isolantes indépendamment de la génération d'agents gonflants utilisés.

### Résumé de l'invention

La présente invention concerne un procédé de récupération et de valorisation d'hydrocarbures fluorés insaturés comprenant les étapes de :
a) la fourniture d'une mousse **M1** constituée de pores renfermant une composition **C1** comprenant au moins un hydrocarbure fluoré insaturé ;
b1) optionnellement le broyage ou la compression de ladite mousse **M1** fournie à l'étape a) pour former une mousse broyée ou une mousse comprimée ;
b2) optionnellement la récupération d'au moins une partie de ladite composition **C1** comprenant au moins un hydrocarbure fluoré insaturé libérée au cours de l'étape b1) ;
c) la dépolymérisation ou la dissolution de ladite mousse **M1** fournie à l'étape a) ou de ladite mousse broyée ou comprimée obtenue à l'étape b1) ;
d) la récupération d'au moins une partie de ladite composition **C1** comprenant au moins un hydrocarbure fluoré insaturé libérée sous forme gazeuse au cours de l'étape c) et optionnellement le mélange de
   celle-ci avec ladite au moins une partie de la composition récupérée à l'étape b2) pour former un flux **A** comprenant au moins un hydrocarbure fluoré insaturé ;
e) la récupération et la séparation dudit flux **A** formé à l'étape d) en une pluralité de flux dont au moins un flux **B1** comprend ledit au moins hydrocarbure fluoré insaturé.

Selon un mode de réalisation préféré, l'hydrocarbure fluoré insaturé comprend trois ou quatre atomes de carbone.

Selon un mode de réalisation préféré, ledit hydrocarbure fluoré insaturé contient une seule double liaison carbone-carbone.

Selon un mode de réalisation préféré, ladite mousse **M1** est constituée de polyuréthane, de polyoléfine, de poly-(méthacrylate de méthyle), de polyhydroxyalcanoate, d'acide polylactique, polyimide, poly(chlorure de vinyle), poly(éthylène/acétate de vinyle), poly(éther-imide), poly(méthacrylimide), polycarbonate ou de polystyrène ou d'un mélange de ceux-ci, de préférence la mousse est constituée de polyuréthane, de polyoléfine, de poly-(méthacrylate de méthyle) ou de polystyrène.

Selon un autre mode de réalisation préféré, ladite mousse **M1** fournie à l'étape a) est constituée de polystyrène ; et l'étape c) est mise en œuvre en présence d'un solvant organique apte à dissoudre le polystyrène ou l'étape c) est mise en œuvre par dépolymérisation du polystyrène par voie thermique.

Selon un mode de réalisation préféré, ladite mousse **M1** fournie à l'étape a) est constituée de polyuréthane et l'étape c) est mise en œuvre par une réaction d'alcoolyse ou d'hydrolyse ou d'ammonolyse ou d'aminolyse.

Dans le cadre de la présente invention, la réaction d'ammonolyse se réfère à la réaction du polyuréthane avec NH₃. La réaction d'aminolyse se réfère à la réaction du polyuréthane ou une amine primaire ou une amine secondaire.

Selon un mode de réalisation préféré, l'étape c) aboutit à la formation de composés polyol, ces derniers étant récupérés, purifiés et recyclés dans un procédé de production de polyuréthane.

Selon un mode de réalisation préféré, l'étape e) de séparation dudit flux est mise en œuvre par distillation, distillation azéotropique, distillation sous pression, distillation extractive, séparation à froid, absorption dans un solvant ou une combinaison de celles-ci.

Selon un mode de réalisation préféré, ledit flux **A** est soumis à une étape d'adsorption avant l'étape e) ou ledit flux **B1** est soumis à une étape d'adsorption après l'étape e).

Selon un mode de réalisation préféré, ledit au moins un hydrocarbure fluoré insaturé compris dans ladite composition **C1** est sélectionné parmi le groupe consistant en 3,3,3-trifluoropropène, 2,3,3,3-tétrafluoropropène, (E/Z)-1,3,3,3-tétrafluoropropène, (E/Z)-1,2,3,3,3-pentafluororpropène, 2-chloro-3,3,3-trifluoropropène, 1-chloro-3,3,3-trifluoropropène, 1,1-dichloro-3,3,3-trifluoropropène et 1,2-dichloro-3,3,3-trifluoropropène, (E/Z)-1,1,1,4,4,4-hexafluorobut-2-ène, 2,4,4,4-tétrafluorobut-1-ène et les mélange de ceux-ci.

Selon un mode de réalisation préféré, ladite composition **C1** comprend également un hydrofluorocarbure différent dudit au moins un hydrocarbure fluoré insaturé, un alcane, du méthyle formate, un gaz inerte, un alcool, un éther, un éther fluoré, un éther fluoré insaturé, une cétone, une fluorocétone, un chlorofluorocarbure ou de l'eau ; ou un mélange de ceux-ci. Selon un mode de réalisation préféré, ledit hydrofluorocarbure différent dudit au moins un hydrocarbure fluoré insaturé est sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea).

Selon un mode de réalisation préféré, ledit chlorofluorocarbure est sélectionné parmi le groupe consistant en trichlorofluorométhane, dichlorodifluorométhane, trichlorotrifluoroéthane, dichlorotétrafluoroéthane, chloropentafluoroéthane.

Selon un mode de réalisation préféré, ledit flux **A** comprend une teneur en ledit au moins un hydrocarbure fluoré comprise entre 50 et 99% en volume sur base du volume total dudit flux **A.** Selon un autre mode de réalisation préféré, l'étape a) comprend également :
- la fourniture d'une mousse **M2** constituée de pores renfermant une composition **C2** comprenant un hydrofluorocarbure sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea), ou
- la fourniture d'une mousse **M3** constituée de pores renfermant une composition **C3** comprenant un chlorofluorocarbure sélectionné parmi le groupe consistant en trichlorofluorométhane, dichlorodifluorométhane, trichlorotrifluoroéthane, dichlorotétrafluoroéthane, chloropentafluoroéthane, ou
- la fourniture d'un mélange desdites mousses **M2** et **M3.**

Selon un mode de réalisation préféré, la mousse M**2** et la mousse **M3** sont constituées d'un polymère identique à celui de ladite mousse **M1.**

Selon un mode de réalisation préféré, la composition **C2** et la composition **C3** sont dépourvues d'hydrocarbure fluoré insaturé.

Selon cet autre mode de réalisation préféré, ledit procédé comprend les étapes :
b1) optionnellement le broyage ou la compression de ladite mousse **M1,** de ladite mousse **M2** et/ou de ladite mousse **M3** fournie à l'étape a) pour former une mousse broyée ou une mousse comprimée ;
b2) optionnellement la récupération d'au moins une partie de ladite composition **C1** et d'au moins une partie de ladite composition **C2** et/ou d'au moins une partie de ladite composition C3, libérée au cours de l'étape b1) ;
c) la dépolymérisation ou la dissolution de ladite mousse **M1** et de ladite mousse **M2** et/ou de ladite mousse **M3** fournie à l'étape a) ou de ladite mousse broyée ou comprimée obtenue à l'étape b1) ;
d) la récupération d'au moins une partie de ladite composition **C1** et d'au moins une partie de ladite composition **C2** et/ou d'au moins une partie de ladite composition **C3**, libérée au cours de l'étape c) et optionnellement leur mélange avec celles récupérée à l'étape b2) pour former un flux **A** ;
e) la récupération et la séparation dudit flux **A** en une pluralité de flux dont au moins un flux **B1** comprend ledit au moins hydrocarbure fluoré insaturé.

Selon cet autre mode de réalisation préféré, ledit flux **A** comprend une teneur en ledit au moins un hydrocarbure fluoré comprise entre 0,1 et 50% en volume sur base du volume total dudit flux **A.**

### Description détaillée de l'invention

La présente invention concerne un procédé de récupération et de valorisation d'hydrocarbures fluorés. Le terme « hydrocarbure fluoré insaturé » désigne un composé hydrocarbure comprenant au moins un atome de fluor sur sa chaine carbonée et au moins une double liaison carbone-carbone. L'hydrocarbure fluoré insaturé peut contenir deux, trois ou quatre atomes de carbone. De préférence, l'hydrocarbure fluoré insaturé comprend trois ou quatre atomes de carbone et une double liaison carbone-carbone.

De préférence, le présent procédé comprend notamment l'étape de :
a) la fourniture d'une mousse **M1** constituée de pores renfermant une composition **C1** comprenant au moins un hydrocarbure fluoré insaturé. Ladite mousse est par exemple issue de dispositifs tels que des réfrigérateurs, des congélateurs, des dispositifs de conservation par le froid, des méthaniers (transport de gaz liquéfié). Comme ceci est décrit dans l'art antérieur, les dispositifs contenant la mousse sont recyclés par l'intermédiaire d'une première étape de démontage desdits dispositifs pour trier les différents constituants. Par exemple, dans des dispositifs réfrigérants, la composition réfrigérante contenue dans le circuit de refroidissement est aspirée pour être recyclée en parallèle. Les différents composants, tels que le moteur, le groupe frigorifique, les interrupteurs, les métaux lourds ferreux ou non ferreux, l'aluminium sont démontés et dirigés ou transportés dans des filières de tri spécifique. Une fois les différents éléments triés, la mousse est récupérée et traitée selon les étapes décrites dans le présent procédé. Ladite mousse est un matériau poreux. Lesdits pores contenus dans la mousse renferment une composition **C1** comprenant au moins un hydrocarbure fluoré insaturé. Cependant, au cours du temps, la composition contenant au moins un hydrocarbure fluoré insaturé reste absorbée par la mousse en tant que telle. La présente invention permet de maximiser la récupération de ladite composition contenant au moins un hydrocarbure fluoré insaturé.

Selon un mode de réalisation préféré, ledit au moins un hydrocarbure fluoré insaturé compris dans ladite composition **C1** est sélectionné parmi le groupe consistant en 3,3,3-trifluoropropène, 2,3,3,3-tétrafluoropropène, (E/Z)-1,3,3,3-tétrafluoropropène, (E/Z)-1,2,3,3,3-pentafluoropropène, 2-chloro-3,3,3-trifluoropropène, 1-chloro-3,3,3-trifluoropropène, 1,1-dichloro-3,3,3-trifluoropropène et 1,2-dichloro-3,3,3-trifluoropropène, (E/Z)-1,1,1,4,4,4-hexafluorobut-2-ène, 2,4,4,4-tétrafluorobut-1-ène et les mélange de ceux-ci. En particulier, ledit hydrocarbure fluoré insaturé est 1-chloro-3,3,3-trifluoropropène.

Selon un mode de réalisation préféré, ladite composition **C1** peut comprendre également un hydrofluorocarbure différent dudit au moins un hydrocarbure fluoré, un alcane, du dioxyde de carbone, du méthyle formate, un gaz inerte, un alcool, un éther, un éther fluoré, un éther fluoré insaturé, une cétone, une fluorocétone, un chlorofluorocarbure ou de l'eau ; ou un mélange de ceux-ci.

Ledit hydrofluorocarbure différent dudit au moins un hydrocarbure fluoré peut être sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea).

De préférence, ledit chlorofluorocarbure est sélectionné parmi le groupe consistant en trichlorofluorométhane, dichlorodifluorométhane, trichlorotrifluoroéthane, dichlorotétrafluoroéthane et chloropentafluoroéthane

Selon un mode de réalisation particulier, le présent procédé peut comprendre les étapes de :
b1) optionnellement le broyage ou la compression de ladite mousse **M1** fournie à l'étape a) pour former une mousse broyée ou comprimée ;
b2) optionnellement la récupération d'au moins une partie de ladite composition **C1** comprenant au moins un hydrocarbure fluoré insaturé libérée au cours de l'étape b1).

Ladite étape b1) peut être mise en œuvre grâce à des broyeurs ou des compresseurs adaptés connus de l'homme du métier. Ledit broyage est mis en œuvre de sorte à obtenir une mousse broyée. Ladite compression est mise en œuvre de sorte à obtenir une mousse comprimée et densifiée. Le broyage ou la compression de la mousse entraine la libération d'au moins une partie de ladite composition comprenant au moins un hydrocarbure fluoré. La mousse broyée peut être de forme et de dimensions variables. Selon un mode de réalisation préféré, la mousse broyée a une dimension comprise entre 0,05 cm et 15 cm. Ladite dimension exprimée ici est la plus grande dimension de ladite mousse broyée. En deçà de 0,05 cm, ladite mousse peut se dégrader et éventuellement se ramollir ou fondre. Au-delà de 15 cm, la libération de ladite composition n'est pas optimale. De préférence, la mousse broyée a une dimension comprise entre 0,1 cm et 14 cm, avantageusement la mousse broyée a une dimension comprise entre 0,1 cm et 13 cm, de préférence la mousse broyée a une dimension comprise entre 0,5 cm et 12 cm, plus préférentiellement la mousse broyée a une dimension comprise entre 1 cm et 11 cm, en particulier la mousse broyée a ont une dimension comprise entre 1 et 5 cm.

Comme mentionné ci-dessus, lors de cette étape b1) de broyage ou de compression, ladite composition **C1** comprenant au moins un hydrocarbure fluoré insaturé contenue dans ladite mousse **M1** va être au moins en partie libérée. Ainsi, lors de l'étape b2), au moins une partie de ladite composition **C1** comprenant au moins un hydrocarbure fluoré insaturé libérée au cours de l'étape b1) est récupérée. Ladite au moins une partie de ladite composition **C1** peut être récupérée par un dispositif d'aspiration. De préférence, lesdites étapes b1) et b2) sont mises en œuvre dans une chambre de traitement hermétiquement fermée et comprenant ledit dispositif d'aspiration. Ce dernier comprend au moins une ou plusieurs buses d'aspiration aptes à récupérer au moins une partie de ladite composition **C1** comprenant au moins un hydrocarbure fluoré insaturé libérée au cours de l'étape de broyage ou de compression b1).

Selon un mode de réalisation particulier, préalablement à la mise en œuvre de l'étape b1) ladite mousse **M1** est mise sous vide partiel. Ceci permet d'évacuer l'air qui l'entoure. De préférence, ladite mousse est mise sous une pression inférieure à 0,9 bar relatif et supérieure à 0,1 bar relatif, de préférence sous une pression inférieure à 0,8 bar relatif et supérieure à 0,2 bar relatif. Ladite au moins une partie de ladite composition **C1** comprenant au moins un hydrocarbure fluoré insaturé récupérée à l'étape b2) peut être épurée. Ladite épuration peut être mise en œuvre par le passage de ladite au moins une partie de ladite composition **C1** comprenant au moins un hydrocarbure fluoré insaturé dans des filtres à poussières. Lesdits filtres à poussières peuvent avoir des pores dont la taille varie entre 0,5 µm et 10 µm. Cette étape d'épuration permet d'éliminer éventuellement des particules ayant été aspirées au même temps que ladite au moins une partie de ladite composition **C1** comprenant au moins un hydrocarbure fluoré insaturé. Subséquemment à cette étape d'épuration, ladite au moins une partie de ladite composition **C1** comprenant au moins un hydrocarbure fluoré insaturé peut être stockée avant d'être traitée ultérieurement comme ceci sera décrit dans la présente demande aux étapes d) et e).

La mousse fournie à l'étape a) et la mousse broyée ou comprimée à l'étape b1) si les étapes b1) et b2) ont été mises en œuvre peuvent être rassemblées pour être traitées selon l'étape c) du présent procédé.

Ledit procédé comprend ainsi une étape c) de :
c) dépolymérisation ou dissolution de la mousse **M1** fournie à l'étape a) et/ou de ladite mousse broyée ou comprimée obtenue à l'étape b1).

Le choix entre une dépolymérisation ou une dissolution dépend du type de mousses à traiter. L'homme du métier de par ses connaissances générales saura reconnaitre la technique la plus adaptée. Par exemple, lorsqu'une mousse de polyuréthane sera traitée à l'étape c), la dépolymérisation sera privilégiée. Au contraire, lorsqu'une mousse de polystyrène sera traitée à l'étape c), la dissolution et la dépolymérisation sont envisageables.

Selon un mode de réalisation préféré, ladite mousse **M1** est constituée de polyuréthane, de polyoléfine, de poly-(méthacrylate de méthyle), de polyhydroxyalcanoate, d'acide polylactique, polyimide, poly(chlorure de vinyle), poly(éthylène/acétate de vinyle), poly(éther-imide), poly(méthacrylimide), polycarbonate ou de polystyrène ou d'un mélange de ceux-ci, de préférence la mousse est constituée de polyuréthane, de polyoléfine, de poly-(méthacrylate de méthyle) ou de polystyrène.

De préférence, ladite mousse est constituée de polyuréthane. La mousse de polyuréthane telle que décrite ici englobe les polymères comprenant uniquement des motifs polyuréthanes et également des copolymères comprenant des motifs polyuréthanes. De préférence, ladite mousse de polyuréthane comprend au moins 20% en poids de motifs polyuréthane sur base du poids total de ladite mousse. Selon un mode de réalisation préféré, ladite mousse de polyuréthane comprend au moins 30% en poids de motifs polyuréthane sur base du poids total de ladite mousse, avantageusement au moins 40% en poids de motifs polyuréthane sur base du poids total de ladite mousse, de préférence au moins 50% en poids de motifs polyuréthane sur base du poids total de ladite mousse, plus préférentiellement au moins 60% en poids de motifs polyuréthane sur base du poids total de ladite mousse, en particulier au moins 70% en poids de motifs polyuréthane sur base du poids total de ladite mousse, plus particulièrement au moins 80% en poids de motifs polyuréthane sur base du poids total de ladite mousse.

Selon la présente invention, le terme « motifs polyuréthane » se réfère à un composé comprenant le motif -[R¹-NH-CO-O-R²]n- dans lequel les substituants R¹ et R² peuvent être sélectionnés parmi le groupe consistant C₁-C₃₀ alkyle, C₂-C₃₀ alkenyle, C₆-C₃₀ aryle ; et n est supérieur à 3, de préférence supérieur à 10, en particulier supérieur à 50. Le terme « alkyle » signifie un groupe alkyle linéaire ou branché contenant au moins 1, et au plus, 30 atomes de carbone. Le groupement alkyle peut être substitué par un groupement aryle non substitué, OH, alkoxy en C₁-C₁₀, un groupement carbonyle ou carboxyle. Le terme « alkényle » signifie un groupe alkényle linéaire ou branché contenant au moins 2, et au plus, 30 atomes de carbone. Le groupe alkényle comprend au moins une double liaison carbone-carbone. Le groupe alkényle peut comprendre deux ou trois doubles liaisons carbone-carbone. Le groupement alkényle peut être substitué par un groupement aryle non substitué, OH, alkoxy en C₁-C₁₀, un groupement carbonyle ou carboxyle. Le terme « aryle » se réfère à un cycle hydrocarboné aromatique contenant le nombre spécifié d'atomes de carbone substitué ou non par un alkyle C₁-C₁₀ non substitué, un groupement carbonyle, carboxyle, OH, alkoxy en C₁-C₁₀.

Lorsque la mousse mise en œuvre dans le procédé est une mousse de polyuréthane, l'étape c) met de préférence en œuvre une étape de dépolymérisation. Ladite dépolymérisation peut être mise en œuvre par des réactions d'alcoolyse, d'hydrolyse, d'ammonolyse ou d'aminolyse.

La réaction d'alcoolyse comprend la mise en contact entre la mousse polyuréthane, broyée ou comprimée ou non, de préférence broyée ou comprimée, avec un alcool. De préférence, l'alcool a un point d'ébullition comprise entre 30°C et 350°C, avantageusement entre 50°C et 325°C, de préférence entre 60°C et 300°C. De préférence, l'alcool est sélectionné parmi le glycérol, le méthanol ou un composé glycol. De préférence, ledit composé glycol a un poids moléculaire inférieur à 1000 g/mol, en particulier inférieur à 500 g/mol. Selon un mode de réalisation préféré, le composé glycol est sélectionné parmi le groupe consistant en éthylène glycol, diéthylène glycol, triéthylène glycol, tetraéthylène glycol, 1,3-propylène glycol, 1,2-propylène glycol, di-, tri-, tétra-1,2-propylène glycol, butane diol et hexane diol. Le ratio molaire entre l'alcool et le motif -[R¹-NH-CO-O-R²]- est compris entre 1/1 et 5/1, avantageusement entre 1/1 et 4/1, de préférence entre 1/1 et 3/1, en particulier entre 1/1 et 2/1. Ladite réaction d'alcoolyse peut être mise en œuvre en présence d'une amine de formule R³ₙNH₃₋ₙ dans laquelle R³ est un radical alkyle comprenant de 1 à 10 atomes de carbone, optionnellement substitué par un ou plusieurs groupements OH ou un ou plusieurs groupements NH2 ; et n est 1 ou 2. Si l'amine comprend plusieurs groupements R³ tels que définis ci-dessus, ceux-ci peuvent être identiques ou différents ou liés entre eux pour former un hétérocycle. De préférence, ladite amine est de formule R³ₙNH₃₋ₙ dans laquelle R³ est un radical alkyle comprenant de 1 à 4 atomes de carbone substitué par un ou plusieurs groupements OH ou un ou plusieurs groupements NH₂ ; n étant tel que défini ci-dessus. En particulier, ladite amine peut être un composé dont le poids moléculaire est inférieur à 200 g/mol, de préférence inférieur à 150 g/mol. Plus particulièrement, ladite amine peut être choisi parmi méthanol amine, éthanol amine, diéthanol amine, dipropanol amine et triéthanol amine et les combinaisons de celles-ci. Alternativement, ladite amine peut être HMTA (i.e. hexaméthylènetétramine). La réaction d'alcoolyse peut également être mise en œuvre en présence d'un catalyseur. Ledit catalyseur peut être un acétate d'un métal alcalin tel que l'acétate de lithium, l'acétate de sodium ou l'acétate de potassium. La quantité de catalyseur peut être comprise entre 0,01% et 5% par rapport au poids de mousse polyuréthane, avantageusement entre 0,1% et 5% en poids, de préférence entre 0,5% et 5% en poids et plus préférentiellement entre 1% et 5% en poids. La mise en œuvre de la dépolymérisation par alcoolyse résulte en la formation de composés polyols et éventuellement de composés uréthane.

La réaction d'hydrolyse comprend la mise en contact entre la mousse polyuréthane, broyée ou comprimée ou non, de préférence broyée ou comprimée, avec de l'eau. L'étape de dépolymérisation de mousse polyuréthane par hydrolyse est de préférence mise en œuvre à haute pression, avantageusement à une pression supérieure à 2 bars absolus, de préférence supérieure à 5 bars absolus, en particulier supérieure à 10 bars absolus. L'étape de dépolymérisation de mousse polyuréthane par hydrolyse est de préférence mise en œuvre à une température de 100°C à 300°C, de préférence de 150°C à 300°C. Ainsi, l'eau est au moins en partie sous la forme de vapeur d'eau. Le temps de réaction pour cette étape d'hydrolyse est de 1 minutes à 5 heures, de préférence de 5 minutes à 2 heures, en particulier de 10 minutes à 1 heure. La mise en œuvre de la dépolymérisation par hydrolyse résulte en la formation de composés polyols, de composé amines et de CO₂.

L'étape de dépolymérisation de mousses polyuréthane peut être mise en œuvre par décomposition en présence de NH₃ (réaction d'ammonolyse), d'une amine primaire ou d'une amine secondaire (réaction d'aminolyse). L'amine primaire ou secondaire est de préférence de formule R⁴R⁵R⁶N dans laquelle R⁴, R⁵ et R⁶ sont indépendamment les uns des autres sélectionnés parmi H, C₁-C₁₀ alkyle substitué ou non par un groupement NH₂ et C₁-C₁₀ alkényle substitué ou non par un groupement NH₂, de préférence H, C₁-C₅ alkyle substitué ou non par un groupement NH₂ et C₁-C₅ alkényle substitué ou non par un groupement NH₂; à condition qu'au moins un des groupements R⁴, R⁵ ou R⁶ représente un hydrogène et que R⁴, R⁵ et R⁶ ne soient simultanément un hydrogène. En particulier, l'amine peut être diéthylènetriamine, triéthylènetétramine. Cette étape peut être mise en œuvre à une température comprise entre 50°C et 300°C, de préférence entre 100°C et 250°C. L'étape de dépolymérisation par réaction avec une amine résulte en la formation de composés polyols et d'amines.

Quelle que soit la méthode utilisée pour l'étape de dépolymérisation des mousses polyuréthane, le présent procédé peut comprendre une étape de récupération et de purification des composés polyols formés au cours de l'étape c). La purification des composés polyols peut être mise en œuvre par distillation, centrifugation ou par extraction en présence d'un solvant. Après purification, de préférence, les composés polyols purifiés sont utilisés dans un procédé de production de polyuréthane.

Selon un autre mode de réalisation préféré, ladite mousse est constituée de polystyrène. Dans ce cas, l'étape c) est mise en œuvre par la mise en contact entre ladite mousse de polystyrène avec un solvant organique apte à solubiliser ladite mousse. Dans la présente invention, le terme « polystyrène » se réfère à un polymère ou un copolymère comprend au moins une unité de formule-[CH₂CH(C₆R⁷₅)]ₙ-dans lequel C₆R⁷₅ représente un cycle carboné aromatique dans lequel R⁷ est indépendamment H ou un alkyle C1-C5 ; et dans lequel n est supérieur à 3, de préférence supérieur à 10, en particulier supérieur à 50. De préférence, cette étape de dissolution peut être mise en œuvre à une température allant de 10°C à 150°C, de préférence de 20°C à 100°C. Selon un mode de réalisation préféré, ledit solvant organique est sélectionné parmi le groupe consistant en n-octane, n-dodecane, cyclohexane, methylcyclohexane, benzène, toluène, naphtalène, styrène, o-xylène, éthylbenzène, p-diéthylbenzène, p-cymène, chlorométhane, 1,1-dichloroéthylène, ethylène dichloride, chloroforme, 1,1-dichloroéthane, trichloroéthylène, tétrachlorure de carbone, chlorobenzène, o-dichlorobenzène, tétrahydrofurane, 1,4-dioxane, dibenzyl éther, acétone, méthyle éthyle cétone, cyclohexanone, diéthylcétone, acétophénone, méthyle isobutyle cétone, méthyle isoamyle cétone, isophorone, di-(isobutyle)cétone, méthyle acétate, éthyle formate, propylène-1,2-carbonate, éthyle acétate, diéthyle carbonate, n-butyle acétate, 2-éthoxyéthyle acétate, isoamyle acétate, 2-nitropropane, nitrobenzène, éthylène diamine, pyridine, morpholine, analine, N-méthyl-2-pyrrolidone, cyclohexylamine, quinoline, N,N-diméthylformamide, carbon disulfide, diméthylsulfoxyde, éthanediol, éthanol, allyle alcool, 1-propanol, 2-propanol, 1-butanol, 2-butanol, benzyl alcool, cyclohexanol, diacétone alcool, éthylène glycol monoethyl éther, diethylène glycol monométhyl éther, diéthylène glycol monoéthyl ether, ethylène glycol monobutyl éther, diéthylène glycol monobutyle éther, 1-décanol, acide benzoïque, acide stéarique, phénol, résorcinol, m-crésol, méthyle salicylate, éthylène glycol, glycérol et propylène glycol ou un mélange de ceux-ci.

La mise en contact entre le solvant organique et la mousse de polystyrène résulte en la formation d'une phase liquide **L** contenant ledit solvant organique et du polystyrène dissous. Un mélange de deux ou plusieurs solvants organiques tels que mentionnés ci-dessus peut être considéré car ladite phase liquide **L** évolue au cours du temps du fait de la dissolution du polystyrène dans celle-ci ; i.e. la phase liquide **L** va se concentrer en polystyrène. Ainsi, un solvant peut être apte à dissoudre le polystyrène lorsque la phase liquide est peu concentrée en celui-ci mais ne plus être apte à dissoudre le polystyrène lorsque la phase liquide devient plus concentrée en polystyrène. Il peut donc être utile d'utiliser un mélange de solvants dont un des solvants permet de dissoudre le polystyrène dans une phase liquide peu concentrée et dont l'autre solvant permet de dissoudre le polystyrène dans une phase liquide plus concentrée. Ainsi, le mélange de solvant organique peut être sélectionné de sorte à permettre une dissolution du polystyrène quelle que soit la concentration en polystyrène dans la phase liquide **L.** Cette phase liquide **L** peut être purifiée ultérieurement. La purification de cette phase liquide **L** inclut par exemple une étape de distillation, de filtration et/ou une précipitation par contact avec un non-solvant. La distillation peut être utilisée lorsqu'un mélange de solvants organiques est utilisé, notamment lorsque les solvants organiques forment un mélange azéotrope. Ce dernier peut être récupéré et recyclé pour être de nouveau mis en contact avec du polystyrène. La filtration permet d'éliminer des résidus insolubles dans ledit solvant organique. La purification peut également comprendre une étape subséquente de précipitation et de lavage. Ainsi, la phase liquide **L** est, de préférence, mise en contact avec un non-solvant apte à faire précipiter le polystyrène contenu dans la phase liquide **L.** Le ou les solvant(s) organiques et le non-solvant sont récupérés, séparés et recyclés. De préférence, le ou les solvant(s) organiques et le non-solvant sont identiques et sont aptes à dissoudre ou à précipiter le polystyrène en fonction des conditions de pression et de température. Il suffira ainsi de faire varier les conditions de pression et de température pour faire précipiter le polystyrène et récupérer le ou les solvant(s) organique(s). Le polystyrène ainsi précipité est ensuite soumis à une ou plusieurs étape(s) de lavage avec ledit non-solvant. Le ratio volumique entre le non-solvant et la phase liquide **L** est de 2 :1 à 4 :1. Ledit non-solvant est de préférence un alcane linéaire ou branché en C₆-C₁₀, en particulier un alcane linéaire ou branché en C₆-C₈. La (les) étape(s) de lavage sont généralement suivie(s) par une étape de séchage dudit polystyrène précipité à une température comprise entre 100°C et 130°C, de préférence entre 115°C to 125°C.

Selon un autre mode de réalisation, ladite mousse constituée de polystyrène peut être dépolymérisée à l'étape c). La dépolymérisation du polystyrène est de préférence mise en œuvre par voie thermique. Ainsi, ladite mousse constituée de polystyrène est de préférence chauffée à une température comprise entre 200°C et 500°C. A une telle température, le polystyrène se trouve à l'état fondu puis se dépolymérise. Selon un mode de réalisation particulier, la dépolymérisation peut être mise en œuvre en présence d'un initiateur radicalaire, avantageusement un initiateur radicalaire de type peroxyde. De préférence, l'initiateur radicalaire est sélectionné parmi le groupe consistant en un peroxyde organique, un peroxyde ou superoxyde inorganique tel que le peroxyde de Baryum (BaO₂), le superoxyde de Potassium (KO₂), le superoxyde de Césium (CsO₂), un percarbonate, un composé peroxyhydraté, leurs sels ainsi que leur mélange. On peut citer comme catalyseur d'initiation à la dépolymérisation le peroxyde d'hydrogène (H₂O₂), l'azobisisobutyronitrile (AIBN), le peroxyhydrate de carbonate de sodium (2Na₂CO₃.3H₂O₂), ou de potassium ou de magnésium ou de calcium, le carbonate d'ammonium peroxyhydraté ((NH₄)₂CO₃.H₂O₂), le peroxyde d'urée (CO(NH₂)₂.H₂O₂), le sulfate de sodium peroxyhydraté (2Na₂SO₄.H₂O₂.2H₂O), les complexes d'H₂O₂ et de sels inorganiques, le peroxyhydrate de poly(vinyl pyrrolidone) polymère (PVP.H₂O₂), les persulfates, les permanganates, les perborates, les peroxyhydrates de sels de phosphates.

Selon un autre mode de réalisation, ladite mousse est constituée de poly-(méthacrylate de méthyle). Le terme « poly-(méthacrylate de méthyle) » se réfère à un polymère comprenant des unités de formule -[CH₂-CH(CH₃)(C(O)-O-CH₃)]n avec n supérieur à 3, de préférence supérieur à 10, en particulier supérieur à 50. L'étape c) peut être mise en œuvre par dépolymérisation par voie thermique, de préférence à une température de 200°C à 500°C. A une telle température, le poly-(méthacrylate de méthyle) se trouve à l'état solide ou fondu. Selon un mode de réalisation particulier, la dépolymérisation peut être mise en œuvre en présence d'un initiateur radicalaire, avantageusement un initiateur radicalaire de type peroxyde. De préférence, l'initiateur radicalaire est sélectionné parmi le groupe consistant en un peroxyde organique, un peroxyde ou superoxyde inorganique tel que le peroxyde de Baryum (BaO₂), le superoxyde de Potassium (KO₂), le superoxyde de Césium (CsO₂), un percarbonate, un composé peroxyhydraté, leurs sels ainsi que leur mélange. On peut citer comme catalyseur d'initiation à la dépolymérisation le peroxyde d'hydrogène (H₂O₂), l'azobisisobutyronitrile (AIBN), le peroxyhydrate de carbonate de sodium (2Na₂CO₃.3H₂O₂), ou de potassium ou de magnésium ou de calcium, le carbonate d'ammonium peroxyhydraté ((NH₄)₂CO₃.H₂O₂), le peroxyde d'urée (CO(NH₂)₂.H₂O₂), le sulfate de sodium peroxyhydraté (2Na₂SO₄.H₂O₂.2H₂O), les complexes d'H₂O₂ et de sels inorganiques, le peroxyhydrate de poly(vinyl pyrrolidone) polymère (PVP.H₂O₂), les persulfates, les permanganates, les perborates, les peroxyhydrates de sels de phosphates.

Alternativement, lorsque ladite mousse est constituée de poly-(méthacrylate de méthyle), l'étape c) peut être mise en œuvre par dissolution de celle-ci dans un solvant organique, apte à dissoudre le poly-(méthacrylate de méthyle). De préférence, ledit solvant organique est le méthacrylate de méthyle. L'utilisation du méthacrylate de méthyle pour dissoudre le poly-(méthacrylate de méthyle) est intéressante car la phase liquide ainsi formée peut être utilisée, après une éventuelle étape de filtration, dans un procédé de production du poly-(méthacrylate de méthyle). Alternativement, ledit solvant organique peut être sélectionné parmi le groupe consistant en toluène, acétone, butanone, cyclohexanone, nitroéthane, chloroforme, dichlorométhane, benzène, chlorobenzène, xylène, méthoxybenzène, diéthylphthalate, acétate de méthoxypropyle, acétate d'éthyle, lactate d'éthyle, acide formique.

De préférence, la dissolution de la mousse de poly-(méthacrylate de méthyle) est mise en œuvre à une température de 20°C à 200°C, de préférence de 25°C à 100°C, de préférence 30 à 80°C. La dissolution peut être mise en œuvre sous pression.

Selon un autre mode de réalisation, ladite mousse est constituée de polyhydroxyalcanoate (i.e. PHA), de préférence polypropiolactone ou poly-3-hydroxypropionate. Dans ce cas, l'étape c) est mise en œuvre par dépolymérisation. De préférence, la dépolymérisation est mise en œuvre par hydrolyse ou par voie thermique. La dépolymérisation par voie thermique est mise en œuvre à une température supérieure à 100°C, avantageusement supérieure à 150°C, de préférence supérieure à 200°C ; et inférieure à 400°C, de préférence inférieure à 300°C. De préférence, la dépolymérisation par voie thermique est mise en œuvre en présence d'un gaz inerte, tel que l'azote, le CO₂ ou l'argon. De préférence, la dépolymérisation par voie thermique est mise en œuvre à une pression inférieure à 1 bar absolu. Alternativement, la dépolymérisation par voie thermique peut être mise en œuvre sous vide partiel. De préférence, la dépolymérisation par voie thermique est mise en œuvre en présence d'un catalyseur de dépolymérisation. De préférence, ledit catalyseur de dépolymérisation est sélectionné parmi le groupe consistant en des catalyseurs acides, tels que des oxydes mixtes, des zéolithes, des alumines, oxydes de titane ou de zirconium dopés par l'un ou plusieurs des éléments P, S, W, B, Nb, Ta.

Alternativement, l'étape c) peut être mise en œuvre par hydrolyse. Dans ce cas, l'étape c) est mise en œuvre à une température de 20°C à 100°C en présence d'eau.

La mise en œuvre de l'étape c) avec les mousses de type polypropiolactone permet la formation d'acide 3-hydroxypropionique qui peut être valorisé ultérieurement en acide acrylique dans un procédé spécifique.

Selon un autre mode de réalisation, ladite mousse est constituée d'acide polylactique, c'est à dire qu'elle contient des unités de formule -[CH(CH₃)-C(O)O-]n avec n supérieur à 3, de préférence supérieur à 10, en particulier supérieur à 50. L'étape c) peut être mise en œuvre par dissolution ou dépolymérisation. La dissolution de l'acide polylactique peut être mise en œuvre en présence d'un solvant organique sélectionné parmi tétrahydrofurane, dioxane, dioxolane, m-crésol, pyridine, N-méthylpyrrolidone, butyrolactone, éthylacétate, carbonate de propylène, acétone, acétonitrile, nitrobenzène, diméthylacétamide dichlorométhane, chloroforme. Alternativement, la dissolution de l'acide polylactique peut être mise en œuvre en présence d'un solvant organique tel qu'un ester d'acide lactique. L'ester d'acide lactique peut être le lactate de méthyle, lactate d'éthyle, lactate d'isopropyle, lactate de butyle, lactate d'hexyle. L'étape de dissolution peut être suivie par une étape de filtration pour éliminer d'éventuellement particules non solubles dans le solvant organique.

La dépolymérisation de l'acide polylactique peut être mise en œuvre par hydrolyse à une température de 80°C à 180°C, de préférence de 100°C à 150°C, en particulier de 120°C à 140°C. La dépolymérisation par hydrolyse peut être mise en œuvre à une pression inférieure à 1 bara ou à une pression de 1 bara à 10 bara. La dépolymérisation par hydrolyse peut être mise en œuvre en présence d'eau ou d'une solution alcaline de NaOH ou KOH. La dépolymérisation par hydrolyse peut éventuellement être mise en œuvre en présence d'un catalyseur tel qu'un acide de Lewis choisi parmi octoate d'étain, lactate d'étain, octoate d'antimoine, octoate de zinc, APTS ou triazabicyclodecène. La dépolymérisation par hydrolyse peut éventuellement être mise en œuvre en présence d'un catalyseur tel qu'un acide de Bronsted.

La dépolymérisation par hydrolyse d'acide polylactique aboutit à la formation d'acide lactique qui peut être valorisé via une utilisation ultérieure dans des procédés de production d'acide polylactique ou encore par déshydratation en acide acrylique, ou conversion en esters.

Selon un autre mode de réalisation, ladite mousse peut être une polyoléfine. De préférence, la polyoléfine est polyéthylène ou polypropylène. Dans ce cas, l'étape c) est mise en œuvre par dissolution dans un solvant organique. L'étape c) peut être mise en œuvre à une température de 100°C à 300°C. Ledit solvant organique peut être le diméthylformamide, diméthyle sulfoxyde, les xylènes, la tétraline, la décaline ou le 1,2,4-trichlorobenzène. Après dissolution, la phase liquide formée par la polyoléfine dissoute dans le solvant organique peut être filtrée pour éventuellement éliminer des particules insolubles. Après filtration, la polyoléfine dissoute dans la phase liquide peut être précipitée selon les techniques connues de l'homme du métier. Selon un autre mode de réalisation, ladite mousse peut être du polyimide, du polycarbonate ou du poly(éther imide). Dans ce cas, la mousse est traitée par dépolymérisation comme décrit ci-dessus pour le polyuréthane.

Selon un autre mode de réalisation, ladite mousse peut être du poly(methacryl imide). Dans ce cas, la mousse est traitée comme décrit ci-dessus pour le poly(méthyl méthacrylate). La dépolymérisation du poly(méthacryl-imide) permet de générer du méthacrylonitrile qui peut être valorisé d'une manière équivalente à celle du méthacrylate de méthyle.

Selon un autre mode de réalisation, ladite mousse peut être du poly(chlorure de vinyle) ou du poly(éthylène/acétate de vinyle). Dans ce cas, la mousse est traitée comme décrit ci-dessus pour une polyoléfine. Dans le cas du poly(chlorure de vinyle), ce dernier peut être récupéré par précipitation par injection de vapeur qui provoque l'évaporation du solvant.

Selon un mode de réalisation alternatif, lorsque ladite mousse est constituée de polyoléfine, polystyrène ou de poly(chlorure de vinyle) ou de poly(éthylène/acétate de vinyle), l'étape c) peut être mise en œuvre par dévolatilisation. Ladite mousse est chauffée à une température telle que ladite mousse se retrouve à l'état fondu. Ladite composition C1 contenue dans les pores de ladite mousse est ainsi libérée à l'état gazeux et récupérée pour être utilisée à l'étape d) du présent procédé.

Au cours de l'étape c), la dépolymérisation ou la dissolution de la mousse va entrainer la libération de ladite composition **C1** comprenant au moins un hydrocarbure fluoré insaturé. Ainsi, le présent procédé comprend l'étape de :
d) la récupération d'au moins une partie de ladite composition **C1** comprenant au moins un hydrocarbure fluoré insaturé libérée au cours de l'étape c) et optionnellement le mélange de celle-ci avec ladite au moins une partie de la composition récupérée à l'étape b2) pour former un flux **A** comprenant au moins un hydrocarbure fluoré insaturé.

Ladite au moins une partie de ladite composition **C1** peut être récupérée par un dispositif d'aspiration. De préférence, lesdites étapes c) et d) sont mises en œuvre dans une chambre de traitement hermétiquement fermée et comprenant ledit dispositif d'aspiration afin de limiter la contamination des gaz de la composition par les gaz de l'air. Ce dernier comprend au moins une ou plusieurs buses d'aspiration aptes à récupérer au moins une partie de ladite composition comprenant au moins un hydrocarbure fluoré relarguée au cours de l'étape c). Ladite au moins une partie de ladite composition **C1** comprenant au moins un hydrocarbure fluoré récupérée à l'étape d) peut être mélangée avec ladite au moins une partie de la composition **C1** récupérée à l'étape b2) pour former un flux **A** comprenant au moins un hydrocarbure fluoré. Ledit flux **A** peut être épuré par le passage dans un ou plusieurs filtres à poussières. Lesdits filtres à poussières peuvent avoir des pores dont la taille varie entre 0,5 µm et 10 µm. Cette étape d'épuration permet d'éliminer éventuellement des particules ayant été aspirées en même temps que ledit flux **A.**

Ledit flux **A** formé à l'étape d) est récupéré et purifié afin de séparer les constituants compris dans celui-ci.

Ainsi, le présent procédé comprend une étape de :
e) récupération et séparation dudit flux **A** formé à l'étape d) en une pluralité de flux dont au moins un flux **B1** comprend ledit au moins hydrocarbure fluoré insaturé.

Ainsi, ledit flux **A** comprend ledit au moins un hydrocarbure fluoré insaturé sélectionné parmi le groupe consistant en 3,3,3-trifluoropropène, 2,3,3,3-tétrafluoropropène, (E/Z)-1,3,3,3-tétrafluoropropène, (E/Z)-1,2,3,3,3-pentafluoropropène, 2-chloro-3,3,3-trifluoropropène, 1-chloro-3,3,3-trifluoropropène, 1,1-dichloro-3,3,3-trifluoropropène et 1,2-dichloro-3,3,3-trifluoropropène, (E/Z)-1,1,1,4,4,4-hexafluorobut-2-ène, 2,4,4,4-tétrafluorobut-1-ène, et les mélange de ceux-ci.

En fonction des constituants de ladite composition **C1,** ledit flux **A** peut également comprendre un hydrofluorocarbure différent dudit au moins un hydrocarbure fluoré insaturé, un alcane, du méthyle formate, un gaz inerte, un alcool, un éther, un éther fluoré, un éther fluoré insaturé, une cétone, une fluorocétone, un chlorofluorocarbure ou de l'eau ; ou un mélange de ceux-ci. De préférence, ledit hydrofluorocarbure différent dudit au moins un hydrocarbure fluoré insaturé est sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea).

De préférence, le gaz inerte est l'azote, l'argon ou le CO₂ ou un mélange de ceux-ci.

De préférence, le chlorofluorocarbure est sélectionné parmi le groupe consistant en trichlorofluorométhane, dichlorodifluorométhane, trichlorotrifluoroéthane, dichlorotétrafluoroéthane et chloropentafluoroéthane.

Selon un mode de réalisation préféré, ledit flux **A** comprend 1-chloro-3,3,3-trifluoropropène et un hydrofluorocarbure différent dudit au moins un hydrocarbure fluoré insaturé sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea). Alternativement, ledit flux **A** comprend 1-chloro-3,3,3-trifluoropropène et un gaz inerte tel que décrit ci-dessus

Alternativement, la teneur en ledit au moins un hydrocarbure fluoré insaturé dans ledit flux **A** est comprise entre 50 % et 99 % en volume sur base du volume total dudit flux **A.** La teneur en ledit au moins un hydrocarbure fluoré insaturé dans ledit flux **A** peut être entre 51 % et 99 % en vol., entre 52 % et 99 % en vol., entre 53 % et 99 % en vol., entre 54 % et 99 % en vol., entre 55 % et 99 % en vol., entre 56 % et 99 % en vol., entre 57 % et 99 % en vol., entre 58 % et 99 % en vol., entre 59 % et 99 % en vol., entre 60 % et 99 % en vol., entre 61 % et 99 % en vol., entre 62 % et 99 % en vol., entre 63 % et 99 % en vol., entre 64 % et 99 % en vol., entre 65 % et 99 % en vol., entre 66 % et 99 % en vol., entre 67 % et 99 % en vol., entre 68 % et 99 % en vol., entre 69 % et 99 % en vol., entre 70 % et 99 % en vol., entre 71 % et 99 % en vol., entre 72 % et 99 % en vol., entre 73 % et 99 % en vol., entre 74 % et 99 % en vol., ou entre 75 % et 99 % en vol.. La teneur en ledit au moins un hydrocarbure fluoré insaturé dans ledit flux **A** peut également être entre 75 % et 98 % en vol., entre 75 % et 97 % en vol., entre 75 % et 96 % en vol., entre 75 % et 95 % en vol., entre 75 % et 94 % en vol., entre 75 % et 93 % en vol., entre 75 % et 92 % en vol., entre 75 % et 91 % en vol., ou entre 75 % et 90 % en vol. sur base du volume total dudit flux **A.** Ledit flux **A** avec les teneurs mentionnées ci-dessus peut être obtenu lorsque le procédé est mis en œuvre à partir de mousses **M1** dont les pores renferment ladite composition **C1**.

Comme mentionné dans la présente demande, le présent procédé permet également de traiter et de recycler des mousses contenant des générations antérieures d'agents gonflants et ceci simultanément au traitement des mousses **M1** comprenant les agents gonflants de nouvelle génération. Ainsi, dans le présent procédé, l'étape a) peut également comprendre :
- la fourniture d'une mousse **M2** constituée de pores renfermant une composition **C2** comprenant un hydrofluorocarbure sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea), ou
- la fourniture d'une mousse **M3** constituée de pores renfermant une composition **C3** comprenant un chlorofluorocarbure sélectionné parmi le groupe consistant en trichlorofluorométhane, dichlorodifluorométhane, trichlorotrifluoroéthane, dichlorotétrafluoroéthane, chloropentafluoroéthane, ou
- la fourniture d'un mélange desdites mousses **M2** et **M3.**

Lorsque l'étape a) fournit des mousses **M2** et/ou **M3** en plus de la mousse **M1,** la mousse **M2** et la mousse **M3** sont constituées d'un polymère identique à celui de ladite mousse **M1.** Ainsi, la mise en œuvre de l'étape c) s'effectue de la même manière en présence des mousses **M2** et/ou **M3** que pour la mousse **M1.**

De préférence, les mousses **M2** et **M3** contiennent des agents gonflants d'anciennes générations. Ainsi, la composition **C2** et la composition **C3** sont dépourvues d'hydrocarbure fluoré insaturé tel que défini dans la présente demande (représentant la nouvelle génération d'agents gonflants). De préférence, la composition **C2** et la composition **C3** sont dépourvues d'hydrocarbure fluoré insaturé comprenant une double liaison carbone-carbone ; en particulier, la composition **C2** et la composition **C3** sont dépourvues d'hydrocarbure fluoré insaturé comprenant trois ou quatre atomes de carbone et une double liaison carbone-carbone. Plus particulièrement, la composition **C2** et la composition **C3** sont dépourvues d'hydrocarbure fluoré insaturé sélectionné parmi le groupe consistant en 3,3,3-trifluoropropène, 2,3,3,3-tétrafluoropropène, (E/Z)-1,3,3,3-tétrafluoropropène, (E/Z)-1,2,3,3,3-pentafluoropropène, 2-chloro-3,3,3-trifluoropropène, 1-chloro-3,3,3-trifluoropropène, 1,1-dichloro-3,3,3-trifluoropropène et 1,2-dichloro-3,3,3-trifluoropropène, (E/Z)-1,1,1,4,4,4-hexafluorobut-2-ène, 2,4,4,4-tétrafluorobut-1-ène et les mélange de ceux-ci. De manière privilégié, la composition **C2** et la composition **C3** sont dépourvues de 1-chloro-3,3,3-trifluoropropène.

Lorsque des mousses **M2** et/ou **M3** ont été fournies à l'étape a), le procédé comprend les étapes b) à e) suivantes :
b1) optionnellement le broyage ou la compression de ladite mousse **M1,** de ladite mousse **M2** et/ou de ladite mousse **M3** fournie à l'étape a) pour former une mousse broyée ou une mousse comprimée ;
b2) optionnellement la récupération d'au moins une partie de ladite composition **C1** et d'au moins une partie de ladite composition **C2** et/ou d'au moins une partie de ladite composition **C3,** libérée au cours de l'étape b1) ;
c) la dépolymérisation ou la dissolution de ladite mousse **M1** et de ladite mousse **M2** et/ou de ladite mousse **M3** fournie à l'étape a) ou de ladite mousse broyée ou comprimée obtenue à l'étape b1) ;
d) la récupération d'au moins une partie de ladite composition **C1** et d'au moins une partie de ladite composition **C2** et/ou d'au moins une partie de ladite composition **C3,** libérée au cours de l'étape c) et optionnellement leur mélange avec celles récupérée à l'étape b2) pour former un flux**A**;
e) la récupération et la séparation dudit flux **A** en une pluralité de flux dont au moins un flux **B1** comprend ledit au moins hydrocarbure fluoré insaturé.

Ainsi, selon un mode de réalisation particulier, le présent procédé comprend les étapes :
a) la fourniture d'une mousse **M1** constituée de pores renfermant une composition **C1** comprenant au moins un hydrocarbure fluoré insaturé ; et
   - la fourniture d'une mousse **M2** constituée de pores renfermant une composition **C2** comprenant un hydrofluorocarbure sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea), ou
   - la fourniture d'une mousse **M3** constituée de pores renfermant une composition **C3** comprenant un chlorofluorocarbure sélectionné parmi le groupe consistant en trichlorofluorométhane, dichlorodifluorométhane, trichlorotrifluoroéthane, dichlorotétrafluoroéthane, chloropentafluoroéthane, ou
   - la fourniture d'un mélange desdites mousses **M2** et **M3** ;
b1) optionnellement le broyage ou la compression de ladite mousse **M1**, de ladite mousse **M2** et/ou de ladite mousse **M3** fournie à l'étape a) pour former une mousse broyée ou une mousse comprimée ;
b2) optionnellement la récupération d'au moins une partie de ladite composition **C1** et d'au moins une partie de ladite composition **C2** et/ou d'au moins une partie de ladite composition **C3,** libérée au cours de l'étape b1) ;
c) la dépolymérisation ou la dissolution de ladite mousse **M1** et de ladite mousse **M2** et/ou de ladite mousse **M3** fournie à l'étape a) ou de ladite mousse broyée ou comprimée obtenue à l'étape b1) ;
d) la récupération d'au moins une partie de ladite composition **C1** et d'au moins une partie de ladite composition **C2** et/ou d'au moins une partie de ladite composition **C3,** libérée au cours de l'étape c) et optionnellement leur mélange avec celles récupérée à l'étape b2) pour former un flux**A** ;
e) la récupération et la séparation dudit flux **A** en une pluralité de flux dont au moins un flux **B1** comprend ledit au moins hydrocarbure fluoré insaturé.

Dans ce mode de réalisation, de préférence, ledit flux **A** comprend une teneur en ledit au moins un hydrocarbure fluoré comprise entre 0,1 et 50% en volume sur base du volume total dudit flux **A.** En particulier, la teneur en ledit au moins un hydrocarbure fluoré insaturé dans ledit flux **A** est comprise entre 0,1 et 50% en volume sur base du volume total dudit flux **A.** La teneur en ledit au moins un hydrocarbure fluoré insaturé dans ledit flux **A** peut être entre 0,5 % et 50 % en vol., entre 1 % et 50 % en vol., entre 2 % et 50 % en vol., entre 3 % et 50 % en vol., entre 4 % et 50 % en vol., entre 5 % et 50 % en vol., entre 6 % et 50 % en vol., entre 7 % et 50 % en vol., entre 8 % et 50 % en vol., entre 9 % et 50 % en vol., entre 10 % et 50 % en vol., entre 11 % et 50 % en vol., entre 12 % et 50 % en vol., entre 13 % et 50 % en vol., entre 14 % et 50 % en vol., entre 15 % et 50 % en vol., entre 16 % et 50 % en vol., entre 17 % et 50 % en vol., entre 18 % et 50 % en vol., entre 19 % et 50 % en vol., entre 20 % et 50 % en vol., entre 21 % et 50 % en vol., entre 22 % et 50 % en vol., entre 23 % et 50 % en vol., entre 24 % et 50 % en vol., ou entre 25 % et 50 % en vol. La teneur en ledit au moins un hydrocarbure fluoré insaturé dans ledit flux **A** peut également être entre 0,1 % et 49 % en vol., entre 0,1 % et 48 % en vol., entre 0,1 % et 47 % en vol., entre 0,1 % et 46 % en vol., entre 0,1 % et 45 % en vol., entre 0,1 % et 44 % en vol., entre 0,1 % et 43 % en vol., entre 0,1 % et 42 % en vol., entre 0,1 % et 41 % en vol., entre 0,1 % et 40 % en vol., entre 0,1 % et 39 % en vol., entre 0,1 % et 38 % en vol., entre 0,1 % et 37 % en vol., entre 0,1 % et 36 % en vol., entre 0,1 % et 35 % en vol., entre 0,1 % et 34 % en vol., entre 0,1 % et 33 % en vol., entre 0,1 % et 32 % en vol., entre 0,1 % et 31 % en vol., entre 0,1 % et 30% en vol., entre 0,1 % et 29 % en vol., entre 0,1 % et 28 % en vol., entre 0,1 % et 27 % en vol., entre 0,1 % et 26 % en vol., entre 0,1 % et 25 % en vol., entre 0,1 % et 24 % en vol., entre 0,1 % et 23 % en vol., entre 0,1 % et 22 % en vol., entre 0,1 % et 21 % en vol., ou entre 0,1 % et 20 % en vol. sur base du volume total dudit flux **A.** Ledit flux **A** avec les teneurs mentionnées ci-dessus peut être obtenu lorsque le procédé est mis en œuvre à partir d'un mélange de mousses, une partie des mousses renfermant des composés autres que ledit hydrocarbure fluoré insaturé, par exemple ledit hydrofluorocarbure différent dudit au moins un hydrocarbure fluoré insaturé tel que défini dans la présente invention ou un chlorofluorocarbure.

Ledit flux **A** est de préférence séparé par distillation, distillation azéotropique, distillation sous pression, distillation extractive, séparation à froid, absorption dans un solvant ou une combinaison de celles-ci. La séparation dudit flux **A** par l'une quelconque des techniques ci-dessus aboutit à la formation d'une pluralité de flux **B** dont au moins un flux **B1** comprend ledit hydrocarbure fluoré insaturé, de préférence comprenant le 1-chloro-3,3,3-trifluoropropène (i.e. HCFO-1233zd).

Dans ce flux **B1,** la teneur molaire en ledit au moins un hydrocarbure fluoré insaturé, de préférence en HCFO-1233zd, est supérieure à la teneur molaire en ledit au moins un hydrocarbure fluoré insaturé, de préférence en HCFO-1233zd, dans ledit flux **A.** Les conditions de température et de pression appliquées pour ces distillations sont telles que la teneur molaire en au moins un ledit hydrocarbure fluoré insaturé, de préférence en HCFO-1233zd, dans ledit flux **B1** est supérieure à 80%, avantageusement supérieure à 85%, de préférence supérieure à 90%, en particulier supérieure à 95%.

L'étape de séparation par distillation comprend de préférence au moins deux colonnes de distillation. La première colonne de distillation permet par exemple d'éliminer une partie des produits ayant un point d'ébullition inférieur à celui dudit au moins un hydrocarbure fluoré insaturé, de préférence inférieur à celui du 1-chloro-3,3,3-trifluoropropène (HCFO-1233zd). La seconde colonne de distillation permet par exemple d'éliminer une partie des produits ayant un point d'ébullition supérieur à celui dudit au moins un hydrocarbure fluoré insaturé, de préférence supérieur à celui du 1-chloro-3,3,3-trifluoropropène. Alternativement, la première colonne de distillation permet par exemple d'éliminer une partie des produits ayant un point d'ébullition supérieur à celui dudit au moins un hydrocarbure fluoré insaturé, de préférence supérieur à celui du 1-chloro-3,3,3-trifluoropropène (HCFO-1233zd). La seconde colonne de distillation permet par exemple d'éliminer une partie des produits ayant un point d'ébullition inférieur à celui dudit au moins un hydrocarbure fluoré insaturé, de préférence inférieur à celui du 1-chloro-3,3,3-trifluoropropène.

Ledit flux **B1** est celui obtenu après ladite au moins seconde distillation.

Comme indiqué ci-dessus, une pluralité de flux est obtenue à l'étape e). Outre le flux **B1,** un flux **B2** comprenant tout ou partie de la composition **C2** et/ou **C3** peut également être obtenu. Les composés contenus dans la composition **C2** et/ou **C3** et récupérés dans le flux **B2** peuvent être valorisés et utilisés comme intermédiaire ou précurseur de synthèse pour la fabrication de composés pharmaceutiques, de composés phytosanitaires, de polymères fluorés ou pour la fabrication d'autres gaz fluorés ayant des applications dans la réfrigération, les fluides de transfert de chaleur ou les agents gonflants (par exemple, le HFC-152a ou le HFC-143a peuvent être utilisés pour la préparation du 1,1-difluoroéthylène).

Comme mentionné ci-dessus, la distillation extractive peut être utilisée pour séparer les constituants présents dans ledit flux **A.** Dans ce cas, ledit flux **A** est mélangé avec un agent d'extraction organique et le mélange résultant est distillé pour former ledit flux **B1** comprenant ledit au moins un hydrocarbure fluoré.

La distillation extractive est particulièrement avantageuse pour séparer ledit hydrocarbure fluoré dudit hydrofluorocarbure différent dudit au moins un hydrocarbure fluoré. Ainsi, ledit flux A comprenant au moins un hydrocarbure fluoré et ledit hydrofluorocarbure différent dudit au moins un hydrocarbure fluoré est mélangé avec ledit agent d'extraction organique. Le mélange résultant est distillé pour former ledit flux **B1** comprenant ledit au moins un hydrocarbure fluoré et un flux **B2** comprenant ledit agent d'extraction organique et ledit hydrofluorocarbure différent dudit au moins un hydrocarbure fluoré.

De préférence, ledit agent d'extraction organique a un point d'ébullition compris entre 10°C et 200°C.

Selon un mode de réalisation préféré, ledit agent d'extraction organique a un facteur de séparation S_{1,2} supérieur ou égal à 1,1, ledit facteur de séparation étant calculé par la formule S_{1,2} = (γ_{1,S}*P1)/(γ_{2,S}*P2) dans laquelle
γ_{1,S} représente le coefficient d'activité dudit au moins un hydrocarbure fluoré dans ledit agent d'extraction organique à dilution infinie,
P1 représente la pression de vapeur saturante dudit au moins un hydrocarbure fluoré,
γ_{2,S} représente le coefficient d'activité dudit hydrofluorocarbure différent dudit au moins un hydrocarbure fluoré et sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea) dans ledit agent d'extraction organique à dilution infinie,
P2 représente la pression de vapeur saturante dudit hydrofluorocarbure différent dudit au moins un hydrocarbure fluoré et sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea) ; avantageusement, le facteur de séparation S_{1,2} peut être supérieur ou égal à 1,2, de préférence supérieur ou égal à 1,4, plus préférentiellement supérieur ou égal à 1,5, en particulier supérieur ou égal à 1,8, plus particulièrement supérieur ou égal à 1,9.

Selon un mode de réalisation préféré, ledit agent d'extraction organique a un facteur de séparation S_{1,2} supérieur ou égal à 1,1, ledit facteur de séparation étant calculé par la formule S_{1,2} = (γ_{1,S}*P1)/(γ_{2,S}*P2) dans laquelle
γ_{1,S} représente le coefficient d'activité d'un des hydrocarbures fluorés sélectionnés parmi le groupe consistant en 3,3,3-trifluoropropène, 2,3,3,3-tétrafluoropropène, (E/Z)-1,3,3,3-tétrafluoropropène, (E/Z)-1,2,3,3,3-pentafluororpropène, 2-chloro-3,3,3-trifluoropropène, 1-chloro-3,3,3-trifluoropropène, 1,1-dichloro-3,3,3-trifluoropropène et 1,2-dichloro-3,3,3-trifluoropropène, (E/Z)-1,1,1,4,4,4-hexafluorobut-2-ène, 2,4,4,4-tétrafluorobut-1-ène et dans ledit agent d'extraction organique à dilution infinie,
P1 représente la pression de vapeur saturante d'un des hydrocarbures fluorés sélectionnés parmi le groupe consistant en 3,3,3-trifluoropropène, 2,3,3,3-tétrafluoropropène, (E/Z)-1,3,3,3-tétrafluoropropène, (E/Z)-1,2,3,3,3-pentafluororpropène, 2-chloro-3,3,3-trifluoropropène, 1-chloro-3,3,3-trifluoropropène, 1,1-dichloro-3,3,3-trifluoropropène et 1,2-dichloro-3,3,3-trifluoropropène, (E/Z)-1,1,1,4,4,4-hexafluorobut-2-ène, 2,4,4,4-tétrafluorobut-1-ène,
γ_{2,S} représente le coefficient d'activité dudit hydrofluorocarbure différent dudit au moins un hydrocarbure fluoré et sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea) dans ledit agent d'extraction organique à dilution infinie,
P2 représente la pression de vapeur saturante dudit hydrofluorocarbure différent dudit au moins un hydrocarbure fluoré et sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea) ; avantageusement, le facteur de séparation S_{1,2} peut être supérieur ou égal à 1,2, de préférence supérieur ou égal à 1,4, plus préférentiellement supérieur ou égal à 1,5, en particulier supérieur ou égal à 1,8, plus particulièrement supérieur ou égal à 1,9.

Le même composé doit être considéré pour les valeurs de γ_{1,S} et de P1. Ainsi, si le coefficient d'activité pris en compte pour γ_{1,S} est celui du 3,3,3-trifluoropropène, P1 représente la pression de vapeur saturante du 3,3,3-trifluoropropène. Le même composé doit être considéré pour les valeurs de γ_{2,S} et de P2. Ainsi, si γ_{2,S} représente le coefficient d'activité du difluorométhane, alors P2 représente la pression de vapeur saturante du difluorométhane.

Selon un mode de réalisation préféré, ledit agent d'extraction organique a un facteur de séparation S_{1,2} supérieur ou égal à 1,1, ledit facteur de séparation étant calculé par la formule S_{1,2} = (γ_{1,S}*P1)/(γ_{2,S}*P2) dans laquelle
γ_{1,S} représente le coefficient d'activité du 1-chloro-3,3,3-trifluoropropène dans ledit agent d'extraction organique à dilution infinie,
P1 représente la pression de vapeur saturante du 1-chloro-3,3,3-trifluoropropène,
γ_{2,S} représente le coefficient d'activité dudit hydrofluorocarbure différent dudit au moins un hydrocarbure fluoré et sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea) dans ledit agent d'extraction organique à dilution infinie,
P2 représente la pression de vapeur saturante dudit hydrofluorocarbure différent dudit au moins un hydrocarbure fluoré et sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea) ; avantageusement, le facteur de séparation S_{1,2} peut être supérieur ou égal à 1,2, de préférence supérieur ou égal à 1,4, plus préférentiellement supérieur ou égal à 1,5, en particulier supérieur ou égal à 1,8, plus particulièrement supérieur ou égal à 1,9.

Dans la présente demande, la pression de vapeur saturante est considérée pour une température de 25°C.

Selon un mode de réalisation préféré, ledit agent d'extraction organique a une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité dudit hydrofluorocarbure différent dudit au moins un hydrocarbure fluoré et sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea) dans ledit agent d'extraction organique à dilution infinie ; avantageusement, la capacité d'absorption C_{2,S} est supérieure ou égale à 0,40, de préférence supérieure ou égale à 0,60, plus préférentiellement supérieure ou égale à 0,80, en particulier supérieure ou égale à 0,90, plus particulièrement supérieure ou égale à 1,0, de manière privilégiée supérieure ou égale à 1,05. Selon un mode de réalisation préféré, ledit agent d'extraction organique est un solvant choisi parmi le groupe consistant en hydrocarbure, hydrohalocarbure, alcool, cétone, amine, ester, éther, aldéhyde, nitrile, carbonate, thioalkyle, amide, hétérocycle. Avantageusement, ledit agent d'extraction organique est un solvant sélectionné parmi le groupe consistant en alcool, cétone, amine, ester et hétérocycle.

Dans le contexte de la distillation extractive, le terme « hydrocarbure » tel qu'utilisé ici se réfère à des composés linéaires ou branchés d'alcane en C₁-C₂₀, cycloalcane en C₃-C₂₀, alcène en C₅-C₂₀, cycloalcène en C₅-C₂₀, arène en C₆-C₁₈. Par exemple, le terme alcane se réfère à des composés de formule CₙH₂ₙ₊₂ dans lequel n est compris entre 1 et 20. Le terme alcane en C₁-C₂₀ englobe par exemple le pentane, hexane, heptane, octane, nonane, décane ou des isomères de ceux-ci. Le terme alcène en C₅-C₂₀ se réfère à des composés hydrocarbonés comprenant une ou plusieurs doubles liaisons carbone-carbone et comprenant de 5 à 20 atomes de carbone. Le terme cycloalcane en C₃-C₂₀ se réfère à un cycle hydrocarboné saturé comprenant de 3 à 20 atomes de carbone. Le terme aryle en C₆-C₁₈ se réfère à des composés hydrocarbonés cycliques et aromatiques comprenant de 6 à 18 atomes de carbone. Le terme cycloalcène en C₅-C₂₀ se réfère à des composés hydrocarbonés cycliques comprenant de 5 à 20 atomes de carbone et comprenant une ou plusieurs doubles liaisons carbone-carbone. Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « alkényle » désigne un radical monovalent de 2 à 20 atomes de carbone et au moins une double liaison carbone-carbone. Le terme « alkynyle » désigne un radical monovalent de 2 à 20 atomes de carbone et au moins une triple liaison carbone-carbone. Le terme « halogène » se réfère à un groupement -F, -Cl, -Br ou -I. Le terme « cycloalkényle » se réfère à un radical monovalent issu d'un cycloalcène comprenant de 3 à 20 atomes de carbone. Les substituants alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ peuvent être substitués ou non par un ou plusieurs substituants -OH, halogène, -NR^{a}C(O)R^{b}, -C(O)NR^{a}R^{b} -CN, -NO₂, -NR^{a}R^{b}, -OR^{a}, -SR^{a}, -CO₂R^{a}, -OC(O)OR^{a}, -OC(O)R^{a}, -C(O)H, -C(O)R^{a}, dans lequel R^{a} et R^{b} sont indépendamment l'un de l'autre hydrogène, alkyle en C₁-C₂₀ non substitué, alkényle en C₂-C₂₀ non substitué, alkynyle en C₂-C₂₀ non substitué, cycloalkyle en C₃-C₂₀ non substitué, cycloalkényle en C₃-C₂₀ non substitué, aryle en C₆-C₁₈ non substitué. Dans les substituants -NR^{a}R^{b}, R^{a} et R^{b} peuvent former avec l'atome d'azote auquel ils sont rattachés un hétérocycle saturé ou insaturé, aromatique ou non, comprenant de 5 à 10 chainons.

Le terme « hydrohalocarbures » se réfère à des composés de formule R^{a}X dans laquelle R^{a} est sélectionné parmi alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ et X représente un atome de chlore, de fluor, de brome ou d'iode. Les substituants alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ peuvent être substitués ou non par un ou plusieurs substituants -OH, halogène, -NR^{a}C(O)R^{b}, -C(O)NR^{a}R^{b} -CN, -NO₂, -NR^{a}R^{b}, -OR^{a}, -SR^{a}, -CO₂R^{a}, - OC(O)OR^{a}, -OC(O)R^{a}, -C(O)H, -C(O)R^{a}, dans lequel R^{a} et R^{b} sont tels que définis ci-dessus.

Le terme « alcool » se réfère à des hydrocarbures ou hydrohalocarbures tels que définis ci-dessus dans lequel au moins un atome d'hydrogène est remplacé par un groupement hydroxyle -OH.

Le terme « cétone » se réfère à des hydrocarbures comprenant au moins un ou plusieurs groupements fonctionnels carbonyle R^{C}-C(O)-R^{d} dans lequel R^{c} et R^{d} sont indépendamment l'un de l'autre un alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ peuvent être substitués ou non par un ou plusieurs substituants -OH, halogène, -NR^{a}C(O)R^{b}, -C(O)NR^{a}R^{b} -CN, -NO₂, -NR^{a}R^{b}, -OR^{a}, -SR^{a}, -CO₂R^{a}, - OC(O)OR^{a}, -OC(O)R^{a}, -C(O)H, -C(O)R^{a}, dans lequel R^{a} et R^{b} sont tels que définis ci-dessus, R^{c} et R^{d} pouvant être liés entre eux pour former avec le groupement carbonyle auquel ils sont rattachés une cétone cyclique comprenant de 4 à 10 chainons, de préférence de 4 à 7 chainons. La cétone cyclique peut également comprendre une ou plusieurs doubles liaisons carbone-carbone. La cétone cyclique peut également être substituée ou non par un ou plusieurs substituants tels que définis ci-dessus.

Le terme « amine » se réfère à des hydrocarbures comprenant au moins un ou plusieurs groupements fonctionnels amine -NR^{c}R^{d} dans lequel R^{c} et R^{d} sont tels que définis ci-dessus, R^{c} et R^{d} pouvant être liés entre eux pour former avec l'atome d'azote auquel ils sont rattachés un hétérocycle aromatique ou non comprenant de 4 à 10 chainons.

Le terme « esters » se réfère à des composés de formule R^{c}-C(O)-O-R^{d} dans lequel R^{c} et R^{d} sont tels que définis ci-dessus, R^{c} et R^{d} pouvant être liés entre eux pour former avec le groupement ester un cycle comprenant de 4 à 20 atomes de carbone.

Le terme « éther » se réfère à des composés de formule R^{c}-O-R^{d} dans lequel R^{c} et R^{d} sont tels que définis ci-dessus, R^{c} et R^{d} pouvant être liés entre eux pour former avec l'atome d'oxygène auquel ils sont rattachés un hétérocycle comprenant de 4 à 20 atomes de carbone.

Le terme « aldéhyde » se réfère à des composés comprenant au moins un ou plusieurs groupements fonctionnels -C(O)-H.

Le terme « nitrile » se réfère à des composés comprenant au moins un ou plusieurs groupements fonctionnels -CN.

Le terme « carbonate » se réfère à des composés de formule R^{c}-O-C(O)-O-R^{d} dans lequel R^{c} et R^{d} sont tels que définis ci-dessus.

Le terme « thioalkyle » concerne des composés de formule R^{c}SR^{d} dans laquelle R^{c} et R^{d} sont tels que définis ci-dessus.

Le terme « amide » concerne des composés de formule R^{c}C(O)NR^{e}R^{d} dans laquelle R^{c} et R^{d} sont tels que définis ci-dessus, R^{e} ayant la même définition que R^{c}, R^{c} et R^{d} pouvant être liés entre eux pour former avec le groupement amide -C(O)N- auquel ils sont rattachés une amide cyclique comprenant de 4 à 10 chainons, de préférence de 4 à 7 chainons. L'amide cyclique peut également comprendre une ou plusieurs doubles liaisons carbone-carbone. L'amide cyclique peut également être substituée ou non par un ou plusieurs substituants tels que définis ci-dessus.

Le terme « hétérocycle » désigne un cycle carboné comprenant de 4 à 10 chainons dont au moins un des chainons est un hétéroatome sélectionné parmi le groupe consistant en O, S, P et N. L'hétérocycle peut comprendre un ou plusieurs double liaison carbone-carbone ou une ou plusieurs double liaison carbone-hétéroatome ou une ou plusieurs double liaison hétéroatome-hétéroatome. De préférence, l'hétérocycle peut comprendre 1, 2, 3, 4 ou 5 hétéroatomes tel que défini ci-dessus. En particulier, l'hétérocycle peut comprendre 1, 2 ou 3 hétéroatomes sélectionné parmi l'oxygène, l'azote ou le soufre. De préférence, l'hétérocycle peut être un cycle carboné comprenant de 4 à 6 chaînons dont 1, 2 ou 3 chaînons sont des hétéroatomes sélectionnés parmi O ou N. L'hétérocycle peut être ou non substitué par un ou plusieurs substituants choisis parmi -OH, halogène, -NR^{a}C(O)R^{b}, -C(O)NR^{a}R^{b} -CN, -NO₂, -NR^{a}R^{b}, -OR^{a}, -SR^{a}, -CO₂R^{a}, -OC(O)OR^{a}, -OC(O)R^{a}, -C(O)H, -C(O)R^{a} dans lequel R^{a} et R^{b} sont tels que définis ci-dessus. De préférence, les hydrocarbures sont sélectionnés parmi le groupe consistant en cyclohexene, 1,3,5-triethylbenzene, 2,4,4-trimethyl-1-pentene, 1-methylcyclohexene, 1,4-dimethylbenzene, styrene, 1,3,5-trimethylbenzene, 1,2,4,5-tetramethylbenzene, 1,3-diethenylbenzene.

De préférence, les hydrohalocarbures sont sélectionnés parmi le groupe consistant en lodomethane, bromoethane, chlorobromomethane, iodoethane, 2-bromopropane, dichlorobromomethane, 2-chloropropane, 2-iodopropane, bromotrichloromethane, trichloroacetaldehyde, 1,2-dibromopropane, 2-bromobutane, 1,2-dichloropropane, 1,1,2-trichloroethane, 1,2,3-trichloropropene, 1,2-dibromoethane, 1-bromopropane, 3-bromopropene, 1-bromo-2-chloroethane, 1,2-dichloroethane, 1-iodopropane, 2-bromopentane, 1-bromo-3-methylbutane, tribromomethane, 1-bromobutane, 1-chloro-3-bromopropane, 1-bromopentane, 1,3-dichloropropane, 1-bromo-3-fluoropropane, 1,2-dibromo-1-fluoroethane, 1-bromo-1,2-difluoroethylene, bromofluoromethane, 1,1,1-trifluoro-2-bromoethane, 1-chloro-3-fluoropropane, 1-chloro-4-fluorobutane, 2-bromo-2-methylpropane, 2-chloro-2-methylpropane, 2-bromo-2-methylbutane, 2,3-dichloro-2-methylbutane, 1-iodobutane, 1,1,2-trichloropropane, 1,3-dichlorobutane, 2,3-dichlorobutane, 1,2,2-trichloropropane, cis-1,3-dichloropropene, trans-1,3-dichloropropene, 1,3-dichloro-trans-2-butene, 1,2-dichloro-2-butene, 2-chloro-2-methylbutane.

De préférence, les alcools sont sélectionnés parmi le groupe consistant en méthanol, éthanol, 2-propanol, 2,2-dimethyl-1-propanol, 2,2,2-trifluoroethanol, tert-butanol, 2,2,3,3-tetraflouro-1-propanol, 2-chloro-1-propanol, propanol, 2-allyloxyethanol, 2-butanol, 2-aminophenol, 2-methyl-2-butanol, 2-ethyl-1-butanol, isobutanol, 3-pentanol, 1-butanol, 1-methoxy2-propanol, 1-(dimethylamino)-2-propanol, 2-methyl-1-pentanol, 3-methyl-3-pentanol, 2-methoxy1-propanol, 1-ethoxy-2-propanol, 4-methyl-2-pentanol, 2-chloroethanol, 1,2-octanediol, 2-(dimethylamino)-ethanol, 3-hexanol, 2-hexanol, 2-ethoxy-1-propanol, 1-pentanol, 2-propoxyethanol, 1-propoxy-2-propanol, 2,2-difluoroethanol, 1,1,1-trifluoro-2-propanol, 4,4,4-trifluorobutanol, 3-fluoropropanol, 2,3-dimethylbutanol, 1-chloro-2-methyl-2-propanol.

De préférence, les cétones sont sélectionnées parmi le groupe consistant en propanone, butanone, 3-pentanone, 2-pentanone, 3,3-dimethyl-2-butanone, 4-methyl-2-pentanone, 2-hexanone, 5-hexen-2-one, 4-methyl-2-hexanone, 1,1,1-trifluoro-2-propanone.

De préférence, les amines sont sélectionnées parmi le groupe consistant en éthylamine, isopropylamine, ethylmethylamine, 2-amino-2-methylpropane, n-propylamine, isopropylmethylamine, diethylamine, 2-butanamine, n-methylpropylamine, 1-butylamine, diisopropylamine, 3-methyl-2-butanamine, 3-pentylamine, n-methylbutylamine, 1-methoxy-2-propanamine, 2-methoxyethanamine, 2-methoxy-1propanamine, n-pentylamine, n-methylhydroxylamine, dipropylamine, 2-ethoxyethanamine, n-methyl-1,2-ethanediamine, pyridine, 1,2-diaminoethane, 1,2-propanediamine, 2-ethylbutylamine, n-ethylethylenediamine, 2-methylpyridine, 4-methyl-2-hexanamine, hexylamine, cyclohexylamine, n-ethyl-2-dimethylaminoethylamine, 1,3-propanediamine, 2-heptanamine, n,n-diethylethylenediamine, 2,6-dimethylpyridine, 4-methylpyridine, n,n'-diethyl-1,2-ethanediamine, dimethylethanolamine, 1,1-diethoxy-n,n-dimethylmethanamine.

De préférence, les esters sont sélectionnés parmi le groupe consistant méthylacétate, ethylacetate, n-propylformate, isopropylacetate, tert-butylacetate, ethylpropionate, sec-butylacetate, diethylcarbonate, n-butylacetate, bromoaceticacidmethylester, methylformate, methylhexanoate, isopropylformate.

De préférence, les éthers sont sélectionnés parmi le groupe consistant en diethylether, 2-ethoxy-propane, methyl-t-butylether, 2,2-dimethoxypropane, 1-ethoxy-2-methylpropane, 1,2-dimethoxyethane, diethoxymethane, 1-ethoxybutane, 1-methoxy-pentane, 1,2-dimethoxypropane, 1,1-diethoxyethane, trimethoxymethane, 2-chloro-1,1-dimethoxyethane, 2,2-diethoxypropane, 1,1-diethoxypropane, 2-methoxyethanol, methoxycyclohexane, chloromethoxymethane, ethoxyethanol, di-n-butylether, diisopropylether, 1-ethoxy-hexane, 1,1,1-triethoxyethane, 1-methoxy-2-acetoxypropane, dimethoxymethane, ethoxy-ethene, di-n-propylether, 2-methoxy-1-propene, 2,2,2-trifluoroethylmethylether, methylcyclopropylether, 2-ethoxy-2-methyl-propane, 2-ethoxy-butane, sec-butyl-tert-butylether, isobutyl-tert-butylether, 1-methoxy-2-methyl-butane, isopropyl-isobutyl-ether.

De préférence, les aldéhydes sont sélectionnés parmi le groupe consistant en acétaldehyde, isobutanal, methylglyoxal, 2-methylbutanal, 2,6-dimethyl-5-heptenal, hexanal, ethanedial.

De préférence, les nitriles sont sélectionnés parmi le groupe consistant en acetonitrile, propionitrile, butyronitrile, valeronitrile, (methyleneamino)acetonitrile.

De préférence, le carbonate est le diéthylcarbonate.

De préférence, les amides incluent l'éthanethioamide.

De préférence, les thioalkyles sont sélectionnés parmi le group consistant en éthanethiol, dimethylsulfide, 2-propanethiol, tert-butylthiol, 3-mercapto-1,2-propanediol, 1-propanethiol, butanethiol, tetrahydrothiophene, 1-pentanethiol, diethylsulfide, 2-butanethiol, 2-methyl-1-propanethiol, 4-methoxy-2-methyl-2-butanethiol.

De préférence, les hétérocycles sont choisis parmi le groupe consistant en n-ethyl-morpholine, 1-methylpiperazine, n-methylmorpholine, 2-methylpyrazine, tetrahydrofurane, 1,3,5-trioxane, dioxane, 1,3-dioxane, piperidine, 2,6-dimethylmorpholine. Le dioxane se réfère au 1,4-dioxane. Le présent procédé peut également comprendre une étape mettant en œuvre la mise en contact dudit flux **A** ou dudit flux **B1** avec un adsorbant. Ledit adsorbant peut être une zéolithe ou un tamis moléculaire ayant une ouverture de pores de diamètre moyen compris entre 3 Angström et 11 Angström, avantageusement entre 4 Angström et 10 Angström, de préférence entre 5 Angström et 10 Angström. L'adsorbant peut aussi contenir des métaux nobles tels que l'argent, platine, palladium, ruthénium, rhodium ; de préférence l'argent. L'adsorbant peut aussi être un polymère et contenir ces métaux, notamment de l'argent. Cette étape peut donc être mise en œuvre après l'étape d) et avant l'étape e) ou après l'étape e). L'étape d'adsorption peut par exemple permettre d'éliminer un ou plusieurs desdits hydrofluorocarbures ou permettre d'éliminer de l'eau éventuellement présente en faible quantité.

Selon un autre mode de réalisation, l'étape e) est mise en œuvre par séparation à froid. Dans ce cas, ledit flux **A** est refroidi à une température telle que ledit au moins un hydrocarbure fluoré insaturé est sous forme liquide et ainsi formé ledit flux **B1**. Ainsi, les autres constituants du flux **A** ayant un point d'ébullition inférieur à celui dudit au moins un hydrocarbure fluoré insaturé sont sous forme gazeuse et sont éliminés par dégazage. Ceci peut être utile pour éliminer des gaz tels que l'oxygène, l'azote ou le dioxyde de carbone éventuellement présent dans le flux **A.** Ledit flux **B1** peut ensuite être réchauffé à une température telle que ledit au moins un hydrocarbure fluoré insaturé est sous forme gazeuse tout en maintenant les autres constituants éventuellement présents dans ledit flux **B1** sous forme liquide. Ledit au moins un hydrocarbure fluoré insaturé est ainsi récupéré avec un degré de pureté élevé.

Selon un autre mode de réalisation, l'étape e) peut être mise en œuvre dans un dispositif de distillation sous pression comprenant au moins un ou plusieurs lit(s) à garnissage rotatif (i.e. rotating packed bed). Dans ce cas, ledit flux **A** est comprimé et éventuellement refroidi à une température telle que ledit au moins un hydrocarbure fluoré est sous forme liquide. Ledit flux **A** ainsi comprimé et éventuellement refroidi est distillé dans un dispositif de distillation sous pression comprenant un ou plusieurs lit(s) à garnissage rotatif pour former et récupérer un flux **B1** tel que défini dans la présente demande. La vitesse dudit au moins un lit à garnissage rotatif est de préférence de 100 à 3000 tpm, avantageusement de 200 à 2500 tpm, de préférence de 500 à 2000 tpm. Dans ce cas, l'étape e) est de préférence mise en œuvre à une pression de 2 à 200 bars absolus, de préférence de 5 à 100 bars absolus.

## Revendications

1. Procédé de récupération et de valorisation d'hydrocarbures fluorés insaturés comprenant les étapes de :
a) la fourniture d'une mousse **M1** constituée de pores renfermant une composition **C1** comprenant au moins un hydrocarbure fluoré insaturé ;
b1) optionnellement le broyage ou la compression de ladite mousse **M1** fournie à l'étape a) pour former une mousse broyée ou une mousse comprimée ;
b2) optionnellement la récupération d'au moins une partie de ladite composition **C1** comprenant au moins un hydrocarbure fluoré insaturé libérée au cours de l'étape b1) ;
c) la dépolymérisation ou la dissolution de ladite mousse **M1** fournie à l'étape a) ou de ladite mousse broyée ou comprimée obtenue à l'étape b1) ;
d) la récupération d'au moins une partie de ladite composition **C1** comprenant au moins un hydrocarbure fluoré insaturé libérée sous forme gazeuse au cours de l'étape c) et optionnellement le mélange de celle-ci avec ladite au moins une partie de la composition récupérée à l'étape b2) pour former un flux **A** comprenant au moins un hydrocarbure fluoré insaturé ;
e) la récupération et la séparation dudit flux **A** formé à l'étape d) en une pluralité de flux dont au moins un flux **B1** comprend ledit au moins hydrocarbure fluoré insaturé.

2. Procédé selon la revendication 1 **caractérisé en ce que** l'hydrocarbure fluoré insaturé comprend trois ou quatre atomes de carbone.

3. Procédé selon l'une quelconque des revendications précédentes **caractérisé en ce que** ledit hydrocarbure fluoré insaturé contient une seule double liaison carbone-carbone.

4. Procédé selon l'une quelconque des revendications précédentes **caractérisé en ce que** ladite mousse **M1** est constituée d'un polymère sélectionné par le groupe consistant en polyuréthane, de polyoléfine, de poly-(méthacrylate de méthyle), de polyhydroxyalcanoate, d'acide polylactique, polyimide, poly(chlorure de vinyle), poly(éthylène/acétate de vinyle), poly(éther-imide), poly(méthacrylimide), polycarbonate ou de polystyrène ou d'un mélange de ceux-ci, de préférence ladite mousse constituée de polyuréthane, de polyoléfine, de poly-(méthacrylate de méthyle) ou de polystyrène.

5. Procédé selon l'une quelconque des revendications précédentes **caractérisé en ce que** ladite mousse **M1** fournie à l'étape a) est constituée de polystyrène ; et l'étape c) est mise en œuvre en présence d'un solvant organique apte à dissoudre le polystyrène ou l'étape c) est mise en œuvre par dépolymérisation du polystyrène par voie thermique.

6. Procédé selon l'une quelconque des revendications précédentes 1 ou 2 **caractérisé en ce que** ladite mousse **M1** fournie à l'étape a) est constituée de polyuréthane et l'étape c) est mise en œuvre par une réaction d'alcoolyse ou d'hydrolyse ou d'ammonolyse ou d'aminolyse.

7. Procédé selon la revendication précédente **caractérisé en ce que** l'étape c) aboutit à la formation de composés polyol, ces derniers étant récupérés, purifiés et recyclés dans un procédé de production de polyuréthane.

8. Procédé selon l'une quelconque des revendications précédentes **caractérisé en ce que** l'étape e) de séparation dudit flux est mise en œuvre par distillation, distillation azéotropique, distillation sous pression, distillation extractive, séparation à froid, absorption dans un solvant ou une combinaison de celles-ci.

9. Procédé selon l'une quelconque des revendications précédentes **caractérisé en ce que** ledit flux **A** est soumis à une étape d'adsorption avant l'étape e) ou ledit flux **B1** est soumis à une étape d'adsorption après l'étape e).

10. Procédé selon l'une quelconque des revendications précédentes **caractérisé en ce que** ledit au moins un hydrocarbure fluoré insaturé compris dans ladite composition **C1** est sélectionné parmi le groupe consistant en 3,3,3-trifluoropropène, 2,3,3,3-tétrafluoropropène, (E/Z)-1,3,3,3-tétrafluoropropène, (E/Z)-1,2,3,3,3-pentafluoropropène, 2-chloro-3,3,3-trifluoropropène, 1-chloro-3,3,3-trifluoropropène, 1,1-dichloro-3,3,3-trifluoropropène et 1,2-dichloro-3,3,3-trifluoropropène, (E/Z)-1,1,1,4,4,4-hexafluorobut-2-ène, 2,4,4,4-tétrafluorobut-1-èneet les mélange de ceux-ci.

11. Procédé selon la revendication précédente **caractérisé en ce que** ladite composition **C1** comprend également un hydrofluorocarbure différent dudit au moins un hydrocarbure fluoré insaturé, un alcane, du méthyle formate, un gaz inerte, un alcool, un éther, un éther fluoré, un éther fluoré insaturé, une cétone, une fluorocétone, un chlorofluorocarbure ou de l'eau ; ou un mélange de ceux-ci.

12. Procédé selon la revendication précédente **caractérisé en ce que** ledit hydrofluorocarbure différent dudit au moins un hydrocarbure fluoré insaturé est sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea).

13. Procédé selon la revendication 11 **caractérisé en ce que** ledit chlorofluorocarbure est sélectionné parmi le groupe consistant en trichlorofluorométhane, dichlorodifluorométhane, trichlorotrifluoroéthane, dichlorotétrafluoroéthane et chloropentafluoroéthane.

14. Procédé selon l'une quelconque des revendications précédentes 1 à 13 **caractérisé en ce que** ledit flux **A** comprend une teneur en ledit au moins un hydrocarbure fluoré comprise entre 50 et 99% en volume sur base du volume total dudit flux **A.**

15. Procédé selon l'une quelconque des revendications précédentes **caractérisé en ce que** l'étape a) comprend également :
- la fourniture d'une mousse **M2** constituée de pores renfermant une composition **C2** comprenant un hydrofluorocarbure sélectionné parmi le groupe consistant en difluorométhane (HFC-32), fluoroéthane (HFC-161), 1,2-difluoroéthane (HFC-152), 1,1-difluoroéthane (HFC-152a), 1,1,2-trifluoroéthane (HFC-143), 1,1,1-trifluoroéthane (HFC-143a), 1,1,2,2-tétrafluoroéthane (HFC-134), 1,1,1,2-tétrafluoroéthane (HFC-134a), 1,1,1,2,2-pentafluoroéthane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea), ou
- la fourniture d'une mousse **M3** constituée de pores renfermant une composition **C3** comprenant un chlorofluorocarbure sélectionné parmi le groupe consistant en trichlorofluorométhane, dichlorodifluorométhane, trichlorotrifluoroéthane, dichlorotétrafluoroéthane, chloropentafluoroéthane, ou
- la fourniture d'un mélange desdites mousses **M2** et **M3.**

16. Procédé selon la revendication précédente **caractérisé en ce que** la mousse **M2** et la mousse **M3** sont constituées d'un polymère identique à celui de ladite mousse **M1.**

17. Procédé selon l'une quelconque des revendications 15 ou 16 **caractérisé en ce que** la composition **C2** et la composition **C3** sont dépourvues d'hydrocarbure fluoré insaturé.

18. Procédé selon l'une quelconque des revendications 15 à 17 **caractérisé en ce qu'**il comprend les étapes :
b1) optionnellement le broyage ou la compression de ladite mousse **M1,** de ladite mousse **M2** et/ou de ladite mousse **M3** fournie à l'étape a) pour former une mousse broyée ou une mousse comprimée ;
b2) optionnellement la récupération d'au moins une partie de ladite composition **C1** et d'au moins une partie de ladite composition **C2** et/ou d'au moins une partie de ladite composition **C3**, libérée au cours de l'étape b1) ;
c) la dépolymérisation ou la dissolution de ladite mousse **M1** et de ladite mousse **M2** et/ou de ladite mousse **M3** fournie à l'étape a) ou de ladite mousse broyée ou comprimée obtenue à l'étape b1) ;
d) la récupération d'au moins une partie de ladite composition **C1** et d'au moins une partie de ladite composition **C2** et/ou d'au moins une partie de ladite composition **C3**, libérée au cours de l'étape c) et optionnellement leur mélange avec celles récupérée à l'étape b2) pour former un flux **A** ;
e) la récupération et la séparation dudit flux **A** en une pluralité de flux dont au moins un flux **B1** comprend ledit au moins hydrocarbure fluoré insaturé.

19. Procédé selon la revendication précédente **caractérisé en ce que** ledit flux **A** comprend une teneur en ledit au moins un hydrocarbure fluoré comprise entre 0,1 et 50% en volume sur base du volume total dudit flux **A.**

## Patentansprüche

1. Verfahren zur Rückgewinnung und Verwertung von ungesättigten fluorierten Kohlenwasserstoffen, umfassend die folgenden Schritte:
a) das Bereitstellen eines Schaums M1, der aus Poren besteht, die eine mindestens einen ungesättigten fluorierten Kohlenwasserstoff umfassende Zusammensetzung C1 enthalten;
b1) optional das Zerkleinern oder Komprimieren des im Schritt a) bereitgestellten Schaums M1, um einen zerkleinerten oder komprimierten Schaum zu bilden;
b2) optional das Rückgewinnen mindestens eines Teils der mindestens einen ungesättigten fluorierten Kohlenwasserstoff umfassenden Zusammensetzung C1, die während des Schritts b1) freigesetzt wird;
c) die Depolymerisation oder das Auflösen des im Schritt a) bereitgestellten Schaums M1 oder des im Schritt b1) erhaltenen zerkleinerten oder komprimierten Schaums;
d) das Rückgewinnen mindestens eines Teils der mindestens einen ungesättigten fluorierten Kohlenwasserstoff umfassenden Zusammensetzung C1, die während des Schritts c) in gasförmiger Form freigesetzt wird, und optional das Mischen von dieser mit dem mindestens einen Teil der im Schritt b2) rückgewonnenen Zusammensetzung, um einen mindestens einen ungesättigten fluorierten Kohlenwasserstoff umfassenden Stoffstrom A zu bilden;
e) das Rückgewinnen und das Trennen des im Schritt d) gebildeten Stoffstroms in eine Mehrzahl von Stoffströmen, unter denen mindestens ein Stoffstrom B1 den mindestens einen ungesättigten fluorierten Kohlenwasserstoff umfasst.

2. Verfahren nach Anspruch 1, **dadurch gekennzeichnet, dass** der ungesättigte fluorierte Kohlenwasserstoff drei oder vier Kohlenstoffatome umfasst.

3. Verfahren nach einem der vorhergehenden Ansprüche, **dadurch gekennzeichnet, dass** der ungesättigte fluorierte Kohlenwasserstoff eine einzige Kohlenstoff-Kohlenstoff-Doppelbindung enthält.

4. Verfahren nach einem der vorhergehenden Ansprüche, **dadurch gekennzeichnet, dass** der Schaum M1 aus einem Polymer besteht, das ausgewählt ist aus der Gruppe bestehend aus Polyurethan, Polyolefin, Polymethylmethacrylat, Polyhydroxyalkanoat, Polymilchsäure, Polyimid, Polyvinylchlorid, Polyethylenvinylacetat, Polyetherimid, Polymethacrylimid, Polycarbonat oder Polystyrol oder einer Mischung von diesen, wobei der Schaum bevorzugt aus Polyurethan, Polyolefin, Polymethylmethacrylat oder Polystyrol besteht.

5. Verfahren nach einem der vorhergehenden Ansprüche, **dadurch gekennzeichnet, dass** der im Schritt a) bereitgestellte Schaum M1 aus Polystyrol besteht; und der Schritt c) in Gegenwart eines organischen Lösungsmittels durchgeführt wird, das geeignet ist, das Polystyrol aufzulösen, oder der Schritt c) durch Depolymerisation des Polystyrols auf thermischem Weg durchgeführt wird.

6. Verfahren nach einem der vorhergehenden Ansprüche 1 oder 2, **dadurch gekennzeichnet, dass** der im Schritt a) bereitgestellte Schaum M1 aus Polyurethan besteht und der Schritt c) durch eine Alkoholyse- oder Hydrolyse- oder Ammonolyse- oder Aminolysereaktion durchgeführt wird.

7. Verfahren nach dem vorhergehenden Anspruch, **dadurch gekennzeichnet, dass** der Schritt c) zur Bildung von Polyolverbindungen führt, wobei Letztere rückgewonnen, gereinigt und in ein Verfahren zur Herstellung von Polyurethan zurückgeführt werden.

8. Verfahren nach einem der vorhergehenden Ansprüche, **dadurch gekennzeichnet, dass** der Schritt e) des Trennens des Stoffstroms durch Destillation, azeotrope Destillation, Druckdestillation, extraktive Destillation, Trennung bei Kälte, Absorption in einem Lösungsmittel oder einer Kombination von diesen durchgeführt wird.

9. Verfahren nach einem der vorhergehenden Ansprüche, **dadurch gekennzeichnet, dass** der Stoffstrom A vor dem Schritt e) einem Adsorptionsschritt unterzogen wird oder der Stoffstrom B1 nach dem Schritt e) einem Adsorptionsschritt unterzogen wird.

10. Verfahren nach einem der vorhergehenden Ansprüche, **dadurch gekennzeichnet, dass** der mindestens eine in der Zusammensetzung C1 enthaltene ungesättigte fluorierte Kohlenwasserstoff ausgewählt ist aus der Gruppe bestehend aus 3,3,3-Trifluorpropen, 2,3,3,3-Tetrafluorpropen, (E/Z)-1,3,3,3-Tetrafluorpropen, (E/Z)-1,2,3,3,3-Pentafluorpropen, 2-Chlor-3,3,3-trifluorpropen, 1-Chlor-3,3,3-trifluorpropen, 1,1-Dichlor-3,3,3-trifluorpropen und 1,2-Dichlor-3,3,3-trifluorpropen, (E/Z)-1,1,1,4,4,4-Hexafluorbut-2-en, 2,4,4,4-Tetrafluorbut-1-en und den Mischungen von diesen.

11. Verfahren nach dem vorhergehenden Anspruch, **dadurch gekennzeichnet, dass** die Zusammensetzung C1 auch einen Fluorkohlenwasserstoff, der von dem mindestens einen ungesättigten fluorierten Kohlenwasserstoff verschieden ist, ein Alkan, Methylformiat, ein inertes Gas, einen Alkohol, einen Ether, einen fluorierten Ether, einen ungesättigten fluorierten Ether, ein Keton, ein Fluorketon, einen Fluorchlorkohlenwasserstoff oder Wasser oder eine Mischung von diesen umfasst.

12. Verfahren nach dem vorhergehenden Anspruch, **dadurch gekennzeichnet, dass** der Fluorkohlenwasserstoff, der verschieden von dem mindestens einen ungesättigten fluorierten Kohlenwasserstoff ist, ausgewählt ist aus der Gruppe bestehend aus Difluormethan (HFC-32), Fluorethan (HFC-161), 1,2-Difluorethan (HFC-152), 1,1-Difluorethan (HFC-152a), 1,1,2-Trifluorethan (HFC-143), 1,1,1-Trifluorethan (HFC-143a), 1,1,2,2-Tetrafluorethan (HFC-134), 1,1,1,2-Tetrafluorethan (HFC-134a), 1,1,1,2,2-Pentafluorethan (HFC-125), 1,1,1,3,3-Pentafluorpropan (HFC-245fa), 1,1,1,3,3,3-Hexafluorpropan (HFC-236fa), 1,1,2,3,3,3-Hexafluorpropan (HFC-236ea), 1,1,1,3,3-Pentafluorbutan (HFC-365mfc), 1,1,1,2,3,3,3-Heptafluorpropan (HFC-227ea).

13. Verfahren nach Anspruch 11, **dadurch gekennzeichnet, dass** der Fluorchlorkohlenwasserstoff ausgewählt ist aus der Gruppe bestehend aus Trichlorfluormethan, Dichlordifluormethan, Trichlortrifluorethan, Dichlortetrafluorethan und Chlorpentafluorethan.

14. Verfahren nach einem der vorhergehenden Ansprüche 1 bis 13, **dadurch gekennzeichnet, dass** der Stoffstrom A einen Gehalt an dem mindestens einen fluorierten Kohlenwasserstoff zwischen 50 und 99 Vol.-% bezogen auf das Gesamtvolumen des Stoffstroms A umfasst.

15. Verfahren nach einem der vorhergehenden Ansprüche, **dadurch gekennzeichnet, dass** der Schritt a) ferner umfasst:
- das Bereitstellen eines Schaums M2, der aus Poren besteht, die eine Zusammensetzung C2 enthalten, die einen Fluorkohlenwasserstoff umfasst, der ausgewählt ist aus der Gruppe bestehend aus Difluormethan (HFC-32), Fluorethan (HFC-161), 1,2-Difluorethan (HFC-152), 1,1-Difluorethan (HFC-152a), 1,1,2-Trifluorethan (HFC-143), 1,1,1-Trifluorethan (HFC-143a), 1,1,2,2-Tetrafluorethan (HFC-134), 1,1,1,2-Tetrafluorethan (HFC-134a), 1,1,1,2,2-Pentafluorethan (HFC-125), 1,1,1,3,3-Pentafluorpropan (HFC-245fa), 1,1,1,3,3,3-Hexafluorpropan (HFC-236fa), 1,1,2,3,3,3-Hexafluorpropan (HFC-236ea), 1,1,1,3,3-Pentafluorbutan (HFC-365mfc), 1,1,1,2,3,3,3-Heptafluorpropan (HFC-227ea), oder
- das Bereitstellen eines Schaums M3, der aus Poren besteht, die eine Zusammensetzung C3 enthalten, die einen Fluorchlorkohlenwasserstoff umfasst, der ausgewählt ist aus der Gruppe bestehend aus Trichlorfluormethan, Dichlordifluormethan, Trichlortrifluorethan, Dichlortetrafluorethan, Chlorpentafluorethan, oder
- das Bereitstellen einer Mischung der Schäume M2 und M3.

16. Verfahren nach dem vorhergehenden Anspruch, **dadurch gekennzeichnet, dass** der Schaum M2 und der Schaum M3 aus einem Polymer bestehen, das identisch mit dem des Schaums M1 ist.

17. Verfahren nach einem der Ansprüche 15 oder 16, **dadurch gekennzeichnet, dass** die Zusammensetzung C2 und die Zusammensetzung C3 frei von ungesättigtem fluoriertem Kohlenwasserstoff sind.

18. Verfahren nach einem der Ansprüche 15 bis 17, **dadurch gekennzeichnet, dass** es die folgenden Schritte umfasst:
b1) optional das Zerkleinern oder Komprimieren des Schaums M1, des Schaums M2 und/oder des Schaums M3, der im Schritt a) bereitgestellt wird, um einen zerkleinerten oder komprimierten Schaum zu bilden;
b2) optional das Rückgewinnen mindestens eines Teils der Zusammensetzung C1 und mindestens eines Teils der Zusammensetzung C2 und/oder mindestens eines Teils der Zusammensetzung C3, die während des Schritts b1) freigesetzt wird;
c) die Depolymerisation oder das Auflösen des Schaums M1 und des Schaums M2 und/oder des Schaums M3, der im Schritt a) bereitgestellt wird, oder des im Schritt b1) erhaltenen zerkleinerten oder komprimierten Schaums;
d) das Rückgewinnen mindestens eines Teils der Zusammensetzung C1 und mindestens eines Teils der Zusammensetzung C2 und/oder mindestens eines Teils der Zusammensetzung C3, die während des Schritts c) freigesetzt wird, und optional ihr Mischen mit denen, die im Schritt b2) rückgewonnen werden, um einen Stoffstrom A zu bilden;
e) das Rückgewinnen und das Trennen des Stoffstroms A in eine Mehrzahl von Stoffströmen, unter denen mindestens ein Stoffstrom B1 den mindestens einen ungesättigten fluorierten Kohlenwasserstoff umfasst.

19. Verfahren nach dem vorhergehenden Anspruch, **dadurch gekennzeichnet, dass** der Stoffstrom A einen Gehalt an dem mindestens einen fluorierten Kohlenwasserstoff zwischen 0,1 und 50 Vol.-% bezogen auf das Gesamtvolumen des Stoffstroms A umfasst.

## Claims

1. Process for recovering and upgrading unsaturated fluorinated hydrocarbons, comprising the steps of:
a) providing a foam M1 consisting of pores containing a composition C1 comprising at least one unsaturated fluorinated hydrocarbon;
b1) optionally grinding or compressing said foam M1 provided in step a) to form a ground foam or a compressed foam;
b2) optionally recovering at least a portion of said composition C1 comprising at least one unsaturated fluorinated hydrocarbon and released during step b1) ;
c) depolymerizing or dissolving said foam M1 provided in step a) or said ground or compressed foam obtained in step b1) ;
d) recovering at least a portion of said composition C1 comprising at least one unsaturated fluorinated hydrocarbon and released in gaseous form during step c) and optionally mixing the latter with said at least a portion of the composition recovered in step b2) to form a stream A comprising at least one unsaturated fluorinated hydrocarbon;
e) recovering and separating said stream A formed in step d) into a plurality of streams of which at least one stream B1 comprises said at least one unsaturated fluorinated hydrocarbon.

2. Process according to Claim 1, **characterized in that** the unsaturated fluorinated hydrocarbon comprises three or four carbon atoms.

3. Process according to either one of the preceding claims, **characterized in that** said unsaturated fluorinated hydrocarbon contains a single carbon-carbon double bond.

4. Process according to any one of the preceding claims, **characterized in that** said foam M1 consists of a polymer selected from the group consisting of polyurethane, polyolefin, poly(methyl methacrylate), polyhydroxyalkanoate, polylactic acid, polyimide, poly(vinyl chloride), poly(ethylene/vinyl acetate), poly(etherimide), poly(methacrylimide), polycarbonate or polystyrene, or a mixture thereof, preferably said foam consists of polyurethane, polyolefin, poly(methyl methacrylate) or polystyrene.

5. Process according to any one of the preceding claims, **characterized in that** said foam M1 provided in step a) consists of polystyrene; and step c) is carried out in the presence of an organic solvent capable of dissolving the polystyrene or step c) is carried out by thermal depolymerization of the polystyrene.

6. Process according to either one of preceding Claims 1 and 2, **characterized in that** said foam M1 provided in step a) consists of polyurethane and step c) is carried out by an alcoholysis or hydrolysis or ammonolysis or aminolysis reaction.

7. Process according to the preceding claim, **characterized in that** step c) results in the formation of polyol compounds, the latter being recovered, purified and recycled into a process for the production of polyurethane.

8. Process according to any one of the preceding claims, **characterized in that** step e) of separating said stream is carried out by distillation, azeotropic distillation, pressurized distillation, extractive distillation, cold separation, absorption in a solvent, or a combination thereof.

9. Process according to any one of the preceding claims, **characterized in that** said stream A is subjected to a step of adsorption prior to step e) or said stream B1 is subjected to a step of adsorption after step e).

10. Process according to any one of the preceding claims, **characterized in that** said at least one unsaturated fluorinated hydrocarbon included in said composition C1 is selected from the group consisting of 3,3,3-trifluoropropene, 2,3,3,3-tetrafluoropropene, (E/Z)-1,3,3,3-tetrafluoropropene, (E/Z)-1,2,3,3,3-pentafluoropropene, 2-chloro-3,3,3-trifluoropropene, 1-chloro-3,3,3-trifluoropropene, 1,1-dichloro-3,3,3-trifluoropropene and 1,2-dichloro-3,3,3-trifluoropropene, (E/Z)-1,1,1,4,4,4-hexafluorobut-2-ene, 2,4,4,4-tetrafluorobut-1-ene, and mixtures thereof.

11. Process according to the preceding claim, **characterized in that** said composition C1 also comprises a hydrofluorocarbon other than said at least one unsaturated fluorinated hydrocarbon, an alkane, methyl formate, an inert gas, an alcohol, an ether, a fluorinated ether, an unsaturated fluorinated ether, a ketone, a fluoroketone, a chlorofluorocarbon or water, or a mixture thereof.

12. Process according to the preceding claim, **characterized in that** said hydrofluorocarbon other than said at least one unsaturated fluorinated hydrocarbon is selected from the group consisting of difluoromethane (HFC-32), fluoroethane (HFC-161), 1,2-difluoroethane (HFC-152), 1,1-difluoroethane (HFC-152a), 1,1,2-trifluoroethane (HFC-143), 1,1,1-trifluoroethane (HFC-143a), 1,1,2,2-tetrafluoroethane (HFC-134), 1,1,1,2-tetrafluoroethane (HFC-134a), 1,1,1,2,2-pentafluoroethane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea).

13. Process according to Claim 11, **characterized in that** said chlorofluorocarbon is selected from the group consisting of trichlorofluoromethane, dichlorodifluoromethane, trichlorotrifluoroethane, dichlorotetrafluoroethane and chloropentafluoroethane.

14. Process according to any one of preceding Claims 1 to 13, **characterized in that** said stream A comprises a content of said at least one fluorinated hydrocarbon of between 50% and 99% by volume, based on the total volume of said stream A.

15. Process according to any one of the preceding claims, **characterized in that** step a) also comprises:
- providing a foam M2 consisting of pores containing a composition C2 comprising a hydrofluorocarbon selected from the group consisting of difluoromethane (HFC-32), fluoroethane (HFC-161), 1,2-difluoroethane (HFC-152), 1,1-difluoroethane (HFC-152a), 1,1,2-trifluoroethane (HFC-143), 1,1,1-trifluoroethane (HFC-143a), 1,1,2,2-tetrafluoroethane (HFC-134), 1,1,1,2-tetrafluoroethane (HFC-134a), 1,1,1,2,2-pentafluoroethane (HFC-125), 1,1,1,3,3-pentafluoropropane (HFC-245fa), 1,1,1,3,3,3-hexafluoropropane (HFC-236fa), 1,1,2,3,3,3-hexafluoropropane (HFC-236ea), 1,1,1,3,3-pentafluorobutane (HFC-365mfc), 1,1,1,2,3,3,3-heptafluoropropane (HFC-227ea), or
- providing a foam M3 consisting of pores containing a composition C3 comprising a chlorofluorocarbon selected from the group consisting of trichlorofluoromethane, dichlorodifluoromethane, trichlorotrifluoroethane, dichlorotetrafluoroethane, chloropentafluoroethane, or
- providing a mixture of said foams M2 and M3.

16. Process according to the preceding claim, **characterized in that** the foam M2 and the foam M3 consist of a polymer identical to that of said foam M1.

17. Process according to either one of Claims 15 and 16, **characterized in that** the composition C2 and the composition C3 are devoid of unsaturated fluorinated hydrocarbon.

18. Process according to any one of Claims 15 to 17, **characterized in that** it comprises the steps of:
b1) optionally grinding or compressing said foam M1, said foam M2 and/or said foam M3 provided in step a) to form a ground foam or a compressed foam;
b2) optionally recovering at least a portion of said composition C1 and at least a portion of said composition C2 and/or at least a portion of said composition C3, released during step b1) ;
c) depolymerizing or dissolving said foam M1 and said foam M2 and/or said foam M3 provided in step a) or said ground or compressed foam obtained in step b1) ;
d) recovering at least a portion of said composition C1 and at least a portion of said composition C2 and/or at least a portion of said composition C3, released during step c), and optionally mixing them with those recovered in step b2) to form a stream A;
e) recovering and separating said stream A into a plurality of streams of which at least one stream B1 comprises said at least one unsaturated fluorinated hydrocarbon.

19. Process according to the preceding claim, **characterized in that** said stream A comprises a content of said at least one fluorinated hydrocarbon of between 0.1% and 50% by volume, based on the total volume of said stream A.
